(19) Europäisches Patentamt
European Patent Office
Office européen des brevets

(11) Veröffentlichungsnummer: **0 339 342 A1**

(12) **EUROPÄISCHE PATENTANMELDUNG**

(21) Anmeldenummer: **89106241.6**

(22) Anmeldetag: **08.04.89**

(51) Int. Cl.4: **C07D 207/50 , C07D 401/04 , A61K 31/40**

(30) Priorität: **23.04.88 DE 3813776**
**11.10.88 IT 2226488**

(43) Veröffentlichungstag der Anmeldung:
**02.11.89 Patentblatt 89/44**

(84) Benannte Vertragsstaaten:
**AT BE CH DE ES FR GB GR IT LI NL SE**

(71) Anmelder: **BAYER AG**

**D-5090 Leverkusen 1 Bayerwerk(DE)**

(72) Erfinder: **Angerbauer, Rolf, Dr.**
**Sterntalerweg 29**
**D-5600 Wuppertal 1(DE)**
Erfinder: **Hübsch, Walter, Dr.**
**Am Eckbusch 43/50**
**D-5600 Wuppertal 1(DE)**
Erfinder: **Fey, Peter, Dr.**
**Ursulastrasse 14**
**D-5600 Wuppertal 1(DE)**
Erfinder: **Bischoff, Hilmar, Dr.**
**Am Rohm 78**
**D-5600 Wuppertal 1(DE)**
Erfinder: **Petzinna, Dieter, Dr.**
**Peter-Berten-Strasse 10**
**D-4000 Duesseldorf 12(DE)**
Erfinder: **Schmidt, Delf, Dr.**
**Am Eckbusch 55 b**
**D-5600 Wuppertal 1(DE)**
Erfinder: **Thomas, Günter, Dr.**
**Via Campogallo 21/14**
**I-20020 Arese (Mi)(IT)**

(54) **N-Substituierte N-Amino-pyrrole.**

(57) N-substituierte N-Amino-pyrrole der Formel

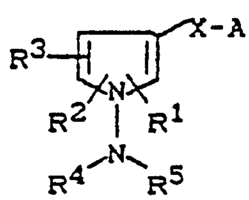

(I)

in welcher die Substituenten, die in dem Text angegebene Bedeutungen haben, und die als Wirkstoffe in

EP 0 339 342 A1

Arzneimittel verwendet werden können.

## N-Substituierte N-Amino-pyrrole

Die Erfindung betrifft substituierte Pyrrole, Zwischenverbindungen zu ihrer Herstellung, ihre Herstellung und ihre Verwendung in Arzneimitteln.

Es ist bekannt, daß bestimmte Inolderivate bzw. Pyrazolderivate Inhibiroten der 3-Hydroxy-3-methylglutaryl Coenzym A Reduktase (HMG-CoA-Reduktase) sind [EP-A 1 114 027; US-Patent 4 613 610].

Es wurden nun substituierte Pyrrole der allgemeinen Formel (I),

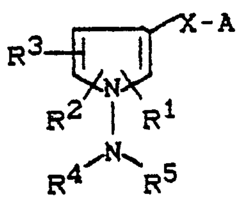

(I)

in welcher

$R^1$ - für Cycloalkyl steht, oder

- für Alkyl steht, das substituiert sein kann durch Halogen, Cyano, Hydroxy, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^6R^7$,

worin

$R^6$, $R^7$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^6R^7$ substituiert sein kann, worin

$R^6$ und $R^7$ die oben angegebene Bedeutung haben,

oder

- für Aryl steht, das bis zu 5-fach gleich oder verschieden substituiert sein kann durch Alkyl, Hydroxyalkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^6R^7$,

worin

$R^6$ und $R^7$ die oben angegebene Bedeutung haben,

$R^3$ - für Wasserstoff oder

- für Cycloalkyl steht, oder

- für Alkyl steht, das substituiert sein kann durch Halogen, Cyano, Hydroxy, Alkoxy, Alkylthio, Alkylsulfonyl, Trifluormethyl, Trifluormethyl, Trifluormethylthio, Trifluormethylsulfonyl, Alkoxycarbonyl, Acyl oder durch eine Gruppe der Formel $-NR^6-R^7$,

worin

$R^6$, $R^7$ - die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

- für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^6R^7$ substituiert sein kann,

worin

$R^6$ und $R^7$ die oben angegebene Bedeutung haben,

oder
- für Aryl steht, das bis zu 5-fach gleich oder verschieden substituiert sein kann durch Alkyl, Hydroxyalkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy,Trifluormethylthio, Alkoxycarbonyl, Sulfamoyl, Dialkylsulfamoyl, Carbamoyl, Dialkylcarbamoyl oder durch eine Gruppe der Formel $-NR^6R^7$,
worin
$R^6$ und $R^7$ die oben angegebene Bedeutung haben,
$R^4$ und $R^5$ gleich oder verschieden sind und für
- Wasserstoff,
- für Cycloalkyl stehen, oder
- für Alkyl stehen, das substituiert sein kann durch Halogen, Hydroxy, Alkoxy, Alkylthio, Acyl oder durch eine Gruppe der Formel $-NR^6R^7$,
worin
$R^6$, $R^7$ - die oben angegebene Bedeutung haben,
oder durch Carbamoyl, Heteroaryl, Aryl, Aryloxy, Arylthio, wobei die Heteroaryl- und Arylreste der letztgenannten Substituenten bis zu 3-fach gleich oder verschieden durch Halogen, Trifluormethyl, Alkyl, Alkoxy, Alkylthio oder Alkylsulfonyl substituiert sein können,
oder
für Heteroaryl steht, das bis zu 2-fach gleich oder verschieden durch Halogen, Alkyl, Alkoxy, Alkylthio, Alkylsulfonyl, Aryl, Trifluormethyl, Alkoxycarbonyl oder durch eine Gruppe der Formel $-NR^6R^7$ substituiert sein kann,
worin
$R^6$ und $R^7$ die oben angegebene Bedeutung haben,
oder
- für Aryl stehen, das bis zu 2-fach gleich oder verschieden substituiert sein kann durch Alkyl, Hydroxyalkyl, Alkoxy, Alkylthio, Alkylsulfonyl, Halogen, Cyano, Nitro, Trifluormethyl, Alkoxycarbonyl, Carbamoyl oder durch eine Gruppe der Formel $-NR^6R^7$,
worin
$R^6$ und $R^7$ die oben angegebene Bedeutung haben,
oder
- $R^4$ und $R^5$ gemeinsam mit dem N-Atom einen 4- bis 8-gliedrigen Heterocyclus bilden, der ein weiteres Heteroatom Y enthalten kann, wobei
Y für S, O oder $N-R^8$ steht, wobei
$R^8$ Alkyl, Aryl, Aralkyl, Acyl, Alkylsulfonyl, Arylsulfonyl bedeuten kann,
wobei dieser Heterocyclus durch $R^9$ substituiert sein kann, wobei
$R^9$ - für Cycloalkyl steht, oder
- für Alkyl steht, das substituiert sein kann durch Halogen, Hydroxy, Alkoxy, Alkylthio, Acyl oder durch eine Gruppe der Formel $-NR^6R^7$,
worin
$R^6$, $R^7$ - die oben angegebene Bedeutung haben,
oder durch Carbamoyl, Heteroaryl, Aryl, Aryloxy, Arylthio, wobei die Heteroaryl- und Arylreste der letztgenannten Substituenten bis zu 2-fach gleich oder verschieden durch Halogen, Trifluormethyl, Alkyl, Alkoxy, Alkylthio oder Alkylsulfonyl substituiert sein können,
oder
- für Heteroaryl steht, das bis zu 2-fach gleich oder verschieden durch Halogen, Alkyl, Alkoxy, Alkylthio, Alkylsulfonyl, Aryl, Trifluormethyl, Alkoxycarbonyl oder durch eine Gruppe der Formel $-NR^6R^7$ substituiert sein kann,
worin
$R^6$ und $R^7$ die oben angegebene Bedeutung haben,
oder
- für Aryl steht, das bis zu 2-fach gleich oder verschieden substituiert sein kann durch Alkyl, Hydroxyalkyl, Alkoxy, Alkylthio, Alkylsulfonyl, Halogen, Cyano, Nitro, Trifluormethyl, Alkoxycarbonyl, Carbamoyl oder durch eine Gruppe der Formel $-NR^6R^7$,
worin
$R^6$ und $R^7$ die oben angegebene Bedeutung haben,
X - für eine Gruppe der Formel $-CH_2-CH_2-$ oder $-CH=CH-$steht,
und
A - für eine Gruppe der Formel

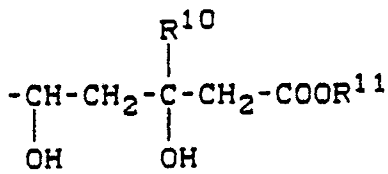 oder 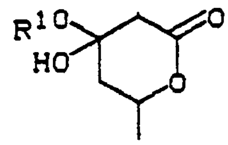 steht,

worin
$R^{10}$ - Wasserstoff oder Alkyl bedeutet
und
$R^{11}$ - Wasserstoff,
- einen Alkyl-, Aryl- oder einen Aralkylrest, oder
- ein Kation bedeutet.

Überraschenderweise zeigen die erfindungsgemäßen substituierten Pyrrole eine gute inhibitorische Wirkung auf die HMG-CoA Reduktase (3-Hydroxy-3-methyl-glutaryl Coenzym A Reduktase).

Cycloalkyl steht im allgemeinen für einen cyclischen Kohlenwasserstoffrest mit 3 bis 8 Kohlenstoffatomen. Bevorzugt ist der Cyclopropyl-, Cyclopentyl- und der Cyclohexylring. Beispielsweise seien Cyclopropyl, Cyclopentyl, Cyclohexyl, Cycloheptyl und Cyclooctyl genannt.

Alkyl steht im allgemeinen für einen geradkettigen oder verzweigten Kohlenwasserstoffrest mit 1 bis 12 Kohlenstoffatomen. Bevorzugt wird Niedrigalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Alkoxy steht im allgemeinen für einen über ein Sauerstoffatom gebundenen geradkettigen oder verzweigten Kohlenwasserstoffrest mit 1 bis 12 Kohlenstoffatomen. Bevorzugt ist Niedrigalkoxy mit 1 bis etwa 6 Kohlenstoffatomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Alkylthio steht im allgemeinen für einen über ein Schwefelatom gebundenen geradkettigen oder verzweigten Kohlenwasserstoffrest mit 1 bis 12 Kohlenstoffatomen. Be vorzugt ist Niedrigalkylthio mit 1 bis etwa 6 Kohlenstoffatomen. Besonders bevorzugt ist ein Alkylthiorest mit 1 bis 4 Kohlenstoffatomen. Beispielsweise seien Methylthio, Ethylthio, Propylthio, Isopropylthio, Butylthio, Isobutylthio, Pentylthio, Isopentylthio, Hexylthio, Isohexylthio, Heptylthio, Isoheptylthio, Octylthio oder Isooctylthio genannt.

Alkylsulfonyl steht im allgemeinen für einen geradkettigen oder verzweigten Kohlenwasserstoffrest mit 1 bis 12 Kohlenstoffatomen, der über eine $SO_2$-Gruppe gebunden ist. Bevorzugt ist Niedrigalkylsulfonyl mit 1 bis etwa 6 Kohlenstoffatomen. Beispielsweise seien genannt: Methylsulfonyl, Ethylsulfonyl, Propylsulfonyl, Isopropylsulfonyl, Butylsulfonyl, Isobutylsulfonyl, Pentylsulfonyl, Isopentylsulfonyl, Hexylsulfonyl, Isohexylsulfonyl.

Aryl steht im allgemeinen für einen aromatischen Rest mit 6 bis etwa 12 Kohlenstoffatomen. Bevorzugte Arylreste sind Phenyl, Naphthyl und Biphenyl.

Aryloxy steht im allgemeinen für einen aromatischen Rest mit 6 bis etwa 12 Kohlenstoffatomen, der über ein Sauerstoffatom gebunden ist. Bevorzugte Aryloxyreste sind Phenoxy oder Naphthyloxy.

Arylthio steht im allgemeinen für einen aromatischen Rest mit 6 bis etwa 12 Kohlenstoffatomen, der über ein Schwefelatom gebunden ist. Bevorzugte Arylthioreste sind Phenylthio oder Naphthylthio.

Arylsulfonyl steht im allgemeinen für einen aromatischen Rest mit 6 bis etwa 12 Kohlenstoffatomen, der über eine $SO_2$-Gruppe gebunden ist. Beispielsweise seien genannt: Phenylsulfonyl, Naphthylsulfonyl und Biphenylsulfonyl.

Aralkyl steht im allgemeinen für einen über eine Alkylenkette gebundenen Arylrest mit 7 bis 14 Kohlenstoffatomen. Bevorzugt werden Aralkylreste mit 1 bis Kohlenstoffatomen im aliphatischen Teil und 6 bis 12 Kohlenstoffatome im aromatischen Teil. Beispielsweise seien folgende Aralkylreste genannt: Benzyl, Naphthylmethyl, Phenethyl und Phenylpropyl.

Aralkoxy steht im allgemeinen für einen Aralkylrest mit 7 bis 14 Kohlenstoffatomen, wobei die Alkylenkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt: Benzyloxy, Naphthylmethoxy, Phenethoxy und Phenylpropoxy.

Aralkylthio steht im allgemeinen für einen Aralkylrest mit 7 bis etwa 14 Kohlenstoffatomen wobei die Alkylkette über ein Schwefelatom gebunden ist. Bevorzugt werden Aralkylthioreste mit 1 bis 6 Kohlenstoffatomen im aliphatischen Teil und 6 bis 12 Kohlenstoffatomen im aromatischen Teil. Beispielsweise seien folgende Aralkylthioreste genannt: Benzylthio, Naphthylmethylthio, Phenethylthio und Phenylpropylthio.

Aralkylsulfonyl steht im allgemeinen für einen Aralkylrest mit 7 bis etwa 14 Kohlenstoffatomen, wobei die Alkylreste über eine $SO_2$-Kette gebunden sind. Bevorzugt werden Aralkylsulfonylreste mit 1 bis 6 Kohlenstoffatomen im aliphatischen Teil und 6 bis 12 Kohlenstoffatomen im aromatischen Teil. Beispielsweise seien folgende Aralkylsulfonylreste genannt: Benzylsulfonyl, Naphthylmethylsulfonyl, Phenethylsulfonyl und Phenylpropylsulfonyl.

Alkoxycarbonyl kann beispielsweise durch die Formel

$$-\underset{\underset{O}{\|}}{C}-OAlkyl$$

dargestellt werden. Alkyl steht hierbei für einen geradkettigen oder verzweigten Kohlenwasserstoffrest mit 1 bis 12 Kohlenstoffatomen. Bevorzugt wird Niedrigalkoxycarbonyl mit 1 bis etwa 6 Kohlenstoffatomen im Alkylteil. Insbesondere bevorzugt wird ein Alkoxycarbonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Acyl steht im allgemeinen für Phenyl oder geradkettiges oder verzweigtes Niedrigalkyl mit 1 bis etwa 6 Kohlenstoffatomen, die über eine Carbonylgruppe gebunden sind. Bevorzugt sind Phenyl und Alkylreste mit bis zu 4 Kohlenstoffatomen. Beispielsweise seien genannt: Benzoyl, Acetyl, Ethylcarbonyl, Propylcarbonyl, Isopropylcarbonyl, Butylcarbonyl und Isobutylcarbonyl.

Halogen steht im allgemeinen für Fluor, Chlor, Brom oder Iod, bevorzugt für Fluor, Chlor oder Brom. Besonders bevorzugt steht Halogen für Fluor oder Chlor.

Heteroaryl im Rahmen der oben angebenen Definition steht im allgemeinen für einen 5- bis 6-gliedrigen aromatischen Ring, der als Heteroatome Sauerstoff, Schwefel und/oder Stickstoff enthalten kann und an den weitere aromatische Ringe ankondensiert sein können. Bevorzugt sind 5- und 6-gliedrige aromatische Ringe, die einen Sauerstoff, ein Schwefel und/oder bis zu 2 Stickstoffatome enthalten und die gegebenenfalls benzokondensiert sind. Als besonders bevorzugte Heteroarylreste seien genannt: Thienyl, Furyl, Pyrolyl, Pyrazolyl, Pyridyl, Pyrimidyl, Pyrazinyl, Pyridazinyl, Chinolyl, Isochinolyl, Chinazolyl, Chinoxalyl, Phthalazinyl, Cinnolyl, Thiazolyl, Benzothiazolyl, Isothiazolyl, Oxazolyl, Benzoxazolyl, Isoxazolyl, Imidazolyl, Benzimidazolyl, Pyrazolyl, Indolyl, und Isoindolyl.

Sulfamoyl (Aminosulfonyl) steht für die Gruppe $SO_2$-$NH_2$.

Falls $R^{11}$ für Alkyl, Aryl, Aralkyl steht, bildet sich ein Ester. Im Rahmen der vorliegenden Erfindung werden physiologisch verträgliche Ester bevorzugt, die in vivo leicht zu einer freien Carboxylgruppe und einem entsprechenden physiologisch verträglichen Alkohol hydrolysiert werden. Hierzu gehören beispielsweise Alkylester ($C_1$ bis $C_4$) und Aralkylester ($C_7$ bis $C_{10}$), bevorzugt Niedrigalkylester sowie Benzylester. Darüber hinaus seien die folgenden Esterreste genannt:
Methylester, Ethylester, Propylester, Benzylester.

Steht $R^{11}$ für ein Kation, so ist bevorzugt ein physiologisch verträgliches Metall- oder Ammoniumkation gemeint. Bevorzugt sind hierbei Alkali- bzw. Erdalkalikationen wie beispielsweise Natrium-, Kalium-, Magnesium- oder Calciumkationen, sowie Aluminium- oder Ammoniumkationen, sowie nicht-toxische substituierte Ammoniumkationen aus Aminen wie Diniedrigalkylamine, Triniedrigalkylamine, Dibenzylamin, N,N′-Dibenzylethylendiamin, N-Benzyl-ß-phenylethylamin, N-Methylmorpholin oder N-Ethylmorpholin, Dihydroabietylamin, N,N′-Bis-dihydroabietylethylendiamin, N-Niedrigalkylpiperidin und andere Amine, die zur Bildung von Salzen verwendet werden können.

Im Rahmen der vorliegenden Erfindung entsprechen die N-substituierten N-Amino-pyrrole (I) der allgemeinen Formel

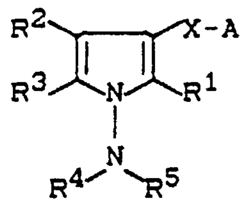

(Ia)

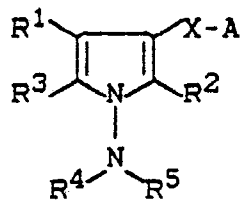

(Ib)

wobei
$R^1$, $R^2$, $R^3$, $R^4$, $R^5$, X und A die oben angegebene Bedeutung haben.

Im Rahmen der allgemeinen Formel (I) sind Verbindungen mit den allgemeinen Formeln (Ia) und (Ib) bevorzugt.

Bevorzugt sind solche Verbindungen der allgemeinen Formel (I)
in welchen
$R^1$ - für Cyclopropyl, Cyclopentyl oder Cyclohexyl steht, oder
- für Niedrigalkyl steht, das substituiert sein kann durch Fluor, Chlor, Brom, Cyano, Hydroxy, Niedrigalkoxy, Niedrigalkylthio, Niedrigalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^6R^7$,
worin
$R^6$ und $R^7$ gleich oder verschieden sind und Niedrigalkyl, Phenyl, Benzyl, Acetyl, Benzoyl, Phenylsulfonyl oder Niederalkylsulfonyl bedeuten,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niedrigalkyl, Niedrigalkoxy, Trifluormethyl oder Trifluormethoxy substituiert sein können,
$R^2$ - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, die bis zu 2-fach gleich oder verschieden substituiert sein können durch Fluor, Chlor, Brom, Niedrigalkyl, Niedrigalkoxy, Phenyl, Phenoxy, Trifluormethyl, Trifluormethoxy oder Niederalkoxycarbonyl, oder
- für Phenyl oder Naphthyl steht, die bis zu 4-fach gleich oder verschieden substituiert sein können durch Niedrigalkyl, Niedrigalkoxy, Niedrigalkylthio, Niedrigalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niedrigalkoxycarbonyl oder durch eine Gruppe der Formel $-NR^6R^7$,
wobei
$R^6$ und $R^7$ die oben angegebene Bedeutung haben,
$R^3$ - für Wasserstoff oder
- für Cyclopropyl, Cyclopentyl oder Cyclohexyl steht oder
- oder für Niedrigalkyl, das substituiert sein kann durch Fluor, Chlor, Cyano, Hydroxy, Niedrigalkoxy, Niedrigalkylthio, Niedrigalkylsulfonyl, Trifluormethyl, Niedrigalkoxycarbonyl, Benzoyl, Niedrigalkylcarbonyl oder durch eine Gruppe der Formel $-NR^6R^7$,
worin
$R^6$ und $R^7$ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylsulfonyl, Benzyloxy, Phenylethoxy, wobei die genannten Heteroaryl- und Arylreste bis zu 2-fach gleich oder verschieden durch Fluor, Chlor, Niedrigalkyl,

Niedrigalkoxy, Trifluormethyl substituiert sein können,
- für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein kann durch Fluor, Chlor, Niedrigalkyl, Niedrigalkoxy, Phenyl, Phenoxy, Trifluormethyl oder Niedrigalkoxycarbonyl, oder
- Phenyl oder Naphthyl bedeutet, das bis zu 2-fach gleich oder verschieden substituiert sein kann durch Niedrigalkyl, Niedrighydroxyalkyl, Niedrigalkoxy, Phenyl, Phenyloxy, Benzyl, Benzyloxy, Phenethyl, Phenylethoxy, Fluor, Chlor, Cyano, Trifluormethyl, Niedrigalkoxycarbonyl oder durch eine Gruppe der Formel $-NR^6R^7$,
wobei
$R^6$ und $R^7$ die oben angegebene Bedeutung haben
$R^4$ und $R^5$ gleich oder verschieden sein können und
- für Wasserstoff,
- für Cyclopropyl, Cyclopentyl oder Cyclohexyl stehen
- oder für Niedrigalkyl stehen, das substituiert sein kann durch Fluor, Chlor, Hydroxy, Niedrigalkoxy, Trifluormethyl, Benzoyl oder durch eine Gruppe der Formel $-NR^6R^7$,
worin
$R^6$ und $R^7$ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Benzyloxy, Phenylethoxy, wobei die genannten Heteroaryl- und Arylreste bis zu 2-fach gleich oder verschieden durch Fluor, Chlor, Niedrigalkyl, Niedrigalkoxy oder Trifluormethyl substituiert sein können,
- für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl stehen, die bis zu 2-fach gleich oder verschieden substituiert sein können durch Fluor, Chlor, Niedrigalkyl, Niedrigalkoxy, Phenyl, Phenoxy, Trifluormethyl oder Niedrigalkoxycarbonyl, oder
- für Phenyl oder Naphthyl stehen, die bis zu 2-fach gleich oder verschieden substituiert sein können durch Niedrigalkyl, Niedrigalkoxy, Niedrigalkylthio, Niedrigalkylsulfonyl, Phenyl, Phenyloxy, Phenylthio, Phenylsulfonyl, Benzyl, Benzyloxy, Phenethyl, Phenylethoxy, Fluor, Chlor, Cyano, Trifluormethyl, Niedrigalkoxycarbonyl oder durch eine Gruppe der Formel $-NR^6R^7$,
wobei
$R^6$ und $R^7$ die oben angegebene Bedeutung haben
oder
$R^4$ und $R^5$ gemeinsam mit dem N-Atom eine 5- bis 7-gliedrigen Heterocyclus bilden, der ein weiteres Heteroatom Y enthalten kann wobei
Y für S, O oder $N-R^8$ steht, wobei
$R^8$ - für Niedrigalkyl, Phenyl, Benzyl, Niedrigalkylsulfonyl, Phenylsulfonyl, Benzoyl, oder Acetyl steht,
wobei dieser Heterocyclus durch $R^9$ substituiert sein kann, wobei
$R^9$ - für Cyclopropyl, Cyclopentyl oder Cyclohexyl steht oder
- für Niedrigalkyl steht, das substituiert sein kann durch Fluor, Chlor, Hydroxy, Niedrigalkoxy, Benzoyl, Niedrigalkylcarbonyl oder durch eine Gruppe der Formel $-NR^6R^7$,
worin
$R^6$, $R^7$ - die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylsulfonyl, Benzyloxy oder Phenylethoxy,
wobei die genannten Heteroaryl- und Arylreste bis zu 2-fach gleich oder verschieden durch Fluor, Chlor, Hydroxyalkyl, Niedrigalkyl, Niedrigalkoxy, Trifluormethyl substituiert sein können,
- für Thienyl, Furyl, Thiazolyl, Isothiazolyl, Oxazolyl, Isoxazolyl, Pyridyl, Pyrimidyl, Pyrazinyl, Pyradazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, die bis zu 2-fach gleich oder verschieden substituiert sein können durch Fluor, Chlor, Niedrigalkyl, Niedrigalkoxy, Phenyl, Phenoxy, Trifluormethyl oder Niedrigalkoxycarbonyl, oder
- für Phenyl steht, das bis zu 2-fach gleich oder verschieden substituiert sein kann durch Niedrigalkyl, Niedrigalkoxy, Niedrigalkylsulfonyl, Phenyl, Phenyloxy, Phenylsulfonyl, Benzyl, Benzyloxy, Phenethyl, Phenylethoxy, Fluor, Chlor, Cyano, Trifluormethyl, Niedrigalkoxycarbonyl oder durch eine Gruppe der Formel $-NR^6R^7$,

wobei

$R^6$ und $R^7$ die oben angegebene Bedeutung haben

X - für eine Gruppe der Formel $-CH_2-CH_2-$ oder $-CH=CH-$steht,

A - für eine Gruppe der Formel

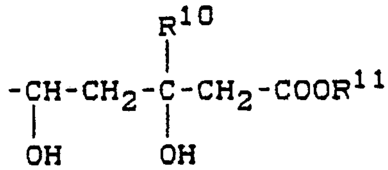 oder 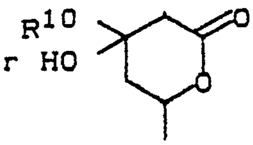 steht,

worin

$R^{10}$ - Wasserstoff oder Niedrigalkyl bedeutet,

und

$R^{11}$- einen $C_1$-$C_6$-Alkylrest, einen $C_6$-$C_{12}$-Alkylrest oder einen $C_7$-$C_{10}$-Alkylrest oder

- ein physiologisch verträgliches Kation bedeutet.

Besonders bevorzugt sind Verbindungen der allgemeinen Formeln (Ia) und (Ib) , in welchen

$R^1$ - für Cyclopropyl, Cyclopentyl oder Cyclohexyl steht, oder

- für Methyl, Ethyl, Propyl, Isopropyl, Butyl, sec.Butyl oder tert.Butyl steht, die substituiert sein können durch Fluor, Chlor, Hydroxy, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylsulfonyl, Benzyloxy, Benzylthio oder Benzylsulfonyl,

$R^2$ - für Pyridyl, Pyrimidyl, Chinolyl oder Isochinolyl steht, die durch Fluor, Chlor, Methyl, Methoxy oder Trifluormethyl substituiert sein können oder

- für Phenyl steht, das bis zu 2-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Methoxycarbonyl, Ethoxycarbonyl, Propoxycarbonyl, Isopropoxycarbonyl, Butoxycarbonyl, Isobutoxycarbonyl oder tert.Butoxycarbonyl,

$R^3$ - Wasserstoff, Cyclopropyl, Cyclopentyl oder Cyclohexyl bedeutet oder

- Methyl, Ethyl, Propyl, Isopropyl, Butyl, Isobutyl, tert.Butyl, Pentyl, Isopentyl, Hexyl oder Isohexyl bedeutet, das substituiert sein kann durch Fluor, Chlor, Cyano, Hydroxy, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe $-NR^6R^7$,

wobei

$R^6$ und $R^7$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,

$R^4$ und $R^5$ gleich oder verschieden sein können und

- für Wasserstoff, Cyclopropyl, Cyclopentyl oder Cyclohexyl stehen, oder

- für Methyl, Ethyl, Propyl, Isopropyl, Butyl, Isobutyl, tert.Butyl, Pentyl, Isopentyl, Hexyl oder Isohexyl stehen, die substituiert sein können durch Fluor, Chlor, Hydroxy, Methoxy, Ethoxy, Methylthio, Ethylthio, Propylthio, Isopropylthio, Butylthio, Isobutylthio, tert.Butylthio, Benzoyl, Acetyl, Ethylcarbonyl oder durch eine Gruppe $-NR^6R^7$,

wobei

$R^6$ und $R^7$ die oben angegebene Bedeutung haben,

oder durch Pyrrolyl, Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.Butoxy, Trifluormethyl substituiert sein können, oder

- für Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzothiazolyl stehen, wobei die genannten Reste durch Fluor Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
- für Phenyl stehen, das bis zu 2-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Phenyl, Phenoxy, Phenylthio, Phenylsulfonyl, Benzyl, Benzyloxy, Fluor, Chlor, Cyano, Hydroxy, Trifluormethyl, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^6R^7$ substituiert sein kann,

wobei

$R^6$ und $R^7$ die oben angegebene Bedeutung haben,

oder

$R^4$ und $R^5$ gemeinsam mit dem N-Atom einen 5- bis 7-gliedrigen Heterocyclus bilden, der ein weiteres Heteroatom Y enthalten kann, wobei

Y für S, O oder $N-R^8$ steht,

wobei $R^8$

Methyl, Ethyl, Propyl, Isopropyl, Butyl, Isobutyl, tert.-Butyl, Phenyl, Benzyl, Acetyl, Methylsulfonyl, Ethylsulfonyl, Propylsulfonyl, Isopropylsulfonyl oder Phenylsulfonyl bedeuten kann

wobei dieser Heterocyclus durch $R^9$ substituiert sein kann

wobei $R^9$

- für Cyclopropyl, Cyclopentyl oder Cyclohexyl steht oder
- für Methyl, Ethyl, Propyl, Isopropyl, Butyl, Isobutyl, tert.-Butyl, Pentyl, Isopentyl, Hexyl oder Isohexyl steht, die substituiert sein können durch Fluor, Chlor, Hydroxy, Methoxy, Ethoxy, Trifluormethyl, Benzoyl, Butyl, Ethylcarbonyl oder durch eine Gruppe $-NR^6R^7$

wobei $R^6$ und $R^7$ die oben angegebene Bedeutung haben

- für Pyridyl, Pyrimidyl oder Benzimidazolyl steht, die bis zu 2-fach gleich oder verschieden substituiert sein können durch Fluor, Chlor, Methyl, Ethyl, Methoxy, Ethoxy, Phenyl, Phenoxy oder Trifluormethyl
- für Phenyl steht, das bis zu 2-fach gleich oder verschieden substituiert sein kann durch Methyl, Ethyl, Propyl, tert.-Butyl, Methylsulfonyl, Ethylsulfonyl, Propylsulfonyl, Phenyl, Phenyloxy, Phenylsulfonyl, Benzyl, Benzyloxy, Phenethyl, Phenylethoxy, Fluor, Chlor, Cyano, Trifluormethyl oder durch eine Gruppe der Formel $-NR^6R^7$,

wobei

$R^6$ und $R^7$ die oben angegebene Bedeutung haben,

X - für eine Gruppe der Formel $-CH_2-CH_2-$ oder $-CH=CH-$steht,

A - für eine Gruppe der Formel

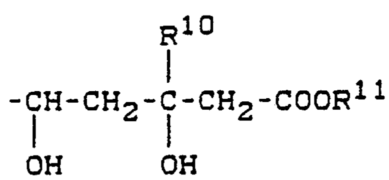 oder 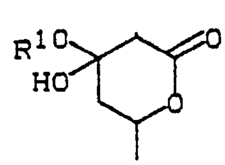 steht,

worin

$R^{10}$ - Wasserstoff, Methyl, Ethyl, Propyl, Isopropyl, Butyl, Isobutyl oder tert.Butyl bedeutet,

und

$R^{11}$- Wasserstoff, Methyl, Ethyl, Propyl, Isopropyl, Butyl, Isobutyl, tert.Butyl oder Benzyl bedeutet oder ein Natrium-, Kalium-, Calcium -, oder Magnesium- oder Ammoniumion bedeutet.

Die erfindungsgemäßen substituierten Pyrrole der allgemeinen Formel (I) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Je nach der Bedeutung der Gruppe X bzw. des Restes A ergeben sich unterschiedliche Stereoisomere, die im folgenden näher erläutert werden sollten:

a) Steht die Gruppe -X- für eine Gruppe der Formel $-CH=CH-$, so können die erfindungsgemäßen Verbindungen in zwei stereoisomeren Formen existieren, die an der Doppelbindung E-konfiguriert (II) oder

Z-konfiguriert (III) sein können:

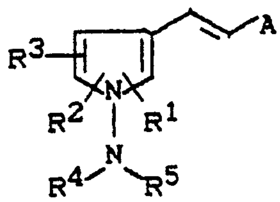

(II) E-Form

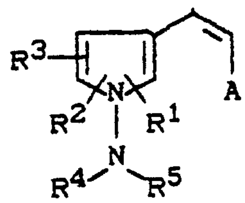

(III) Z-Form

wobei
$R^1$, $R^2$, $R^3$, $R^4$, $R^5$ und A die oben angegebene Bedeutung haben.

Bevorzugt sind diejenigen Verbindungen der allgemeinen Formel (I) die E-konfiguriert sind (II).

b) Steht der Rest -A- für eine Gruppe der Formel

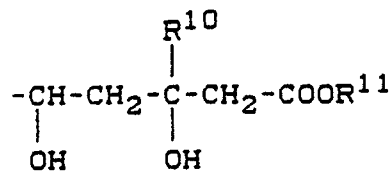

wobei
$R^{10}$ und $R^{11}$ die oben angegebene Bedeutung haben,
so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen.

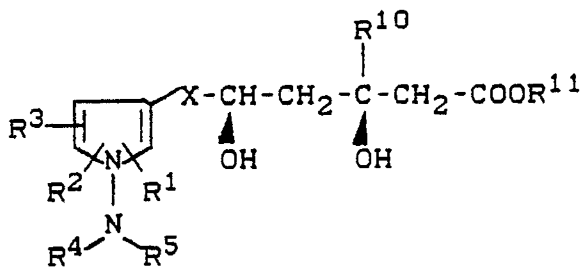

Erythro-Form (IV)

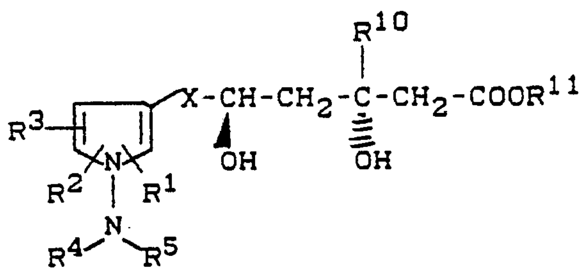

Threo-Form (V)

Sowohl von den Verbindungen in Erythro- als auch in Threo-Konfiguration existieren wiederum jeweils zwei Enantiomere, nämlich 3R,5S-Isomeres bzw. 3S-5R-Isomeres (Erythro-Form) sowie 3R,5R-Isomeres und

3S,5S-Isomeres (Threo-Form).
Bevorzugt sind hierbei die Erythro-konfigurierten Isomeren, besonders bevorzugt das 3R,5S-Isomere sowie das 3R,5S-3S,5R-Racemat.

c) Steht der Rest -A- für eine Gruppe der Formel

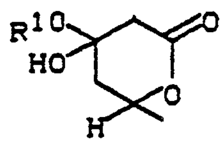

so besitzen die N-substituierten Pyrrole mindestens zwei asymmetrische Kohlenstoffatome, nämlich das Kohlenstoffatom an dem die Hydroxygruppe gebunden ist, und das Kohlenstoffatom an welchem der Rest der Formel

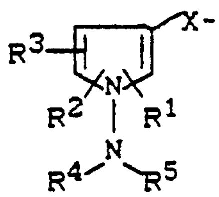

gebunden ist. Je nach der Stellung der Hydroxygruppe zur freien Valenz am Lactonring können die substituierten Pyrrole als cis-Lactone (VI) oder als trans-Lactone (VII) vorliegen.

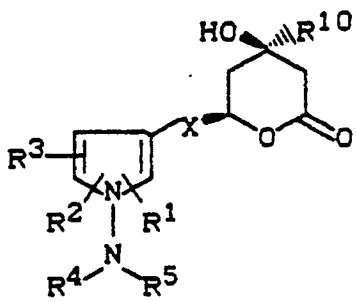

cis-Lacton (VI)

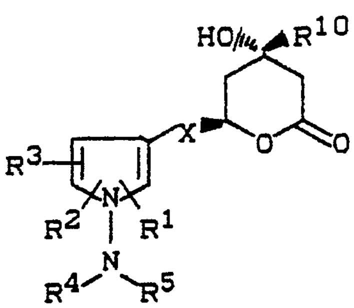

trans-Lacton (VII)

Sowohl vom cis-Lacton aus als auch vom trans-Lacton existieren wiederum jeweils zwei Isomeren nämlich das 4R,6R-Isomere bzw. das 4S,6S-Isomere (cis-Lacton), sowie das 4R,6S-Isomere bzw. 4S,6R-Isomere (trans-Lacton). Bevorzugte Isomere, sind die trans-Lactone. Besonders bevorzugt ist hierbei das 4R,6S-Isomere (trans) sowie das 4R,6S-4S,6R-Racemat.
Beispielsweise seien die folgenden isomeren Formen der substituierten Pyrrole genannt:

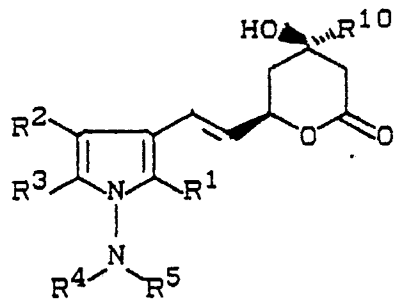

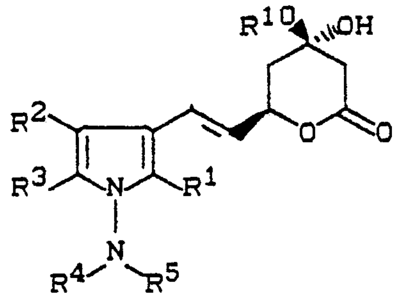

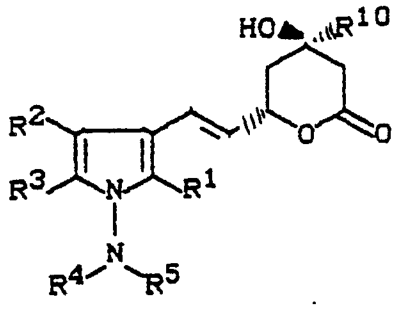

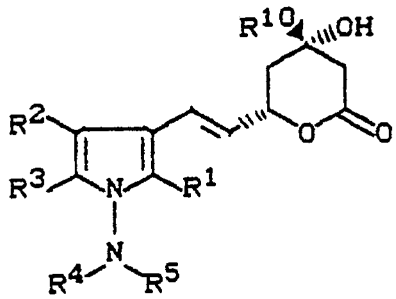

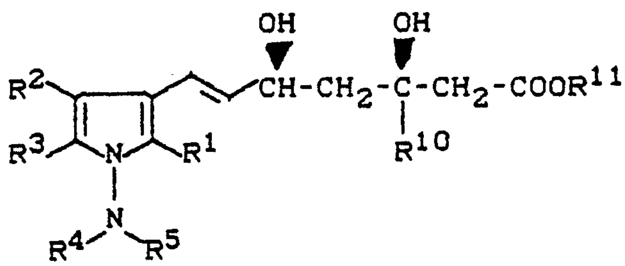

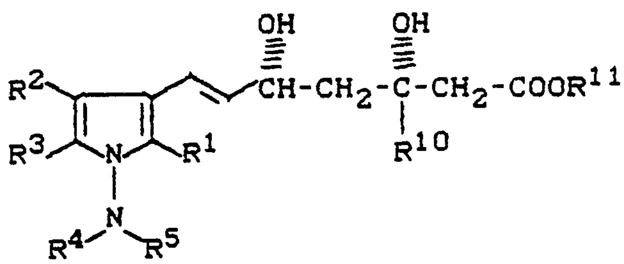

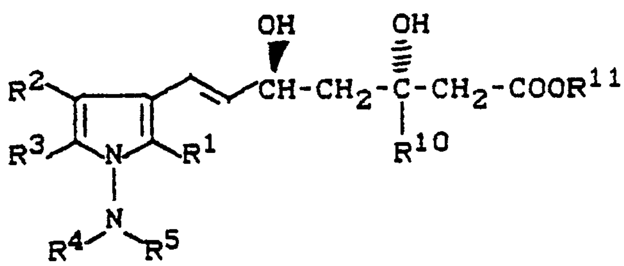

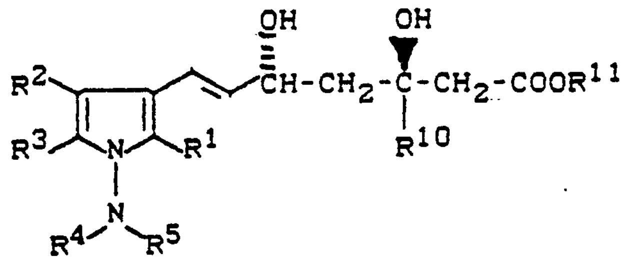

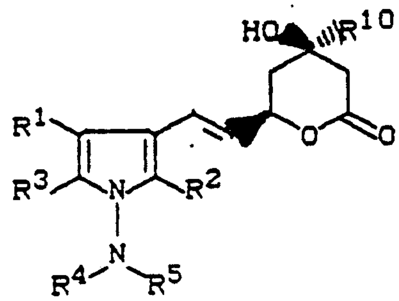

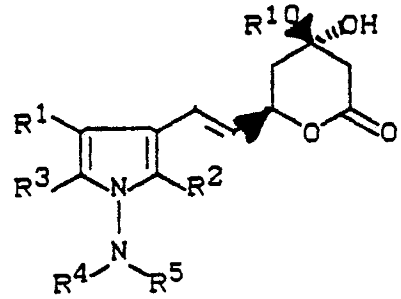

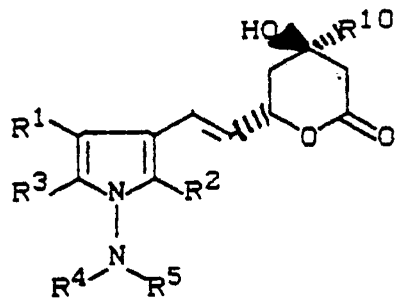

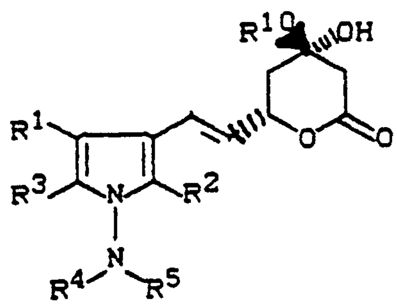

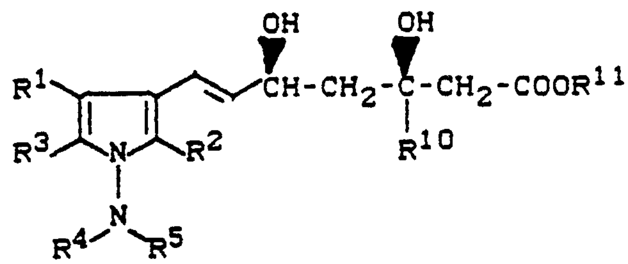

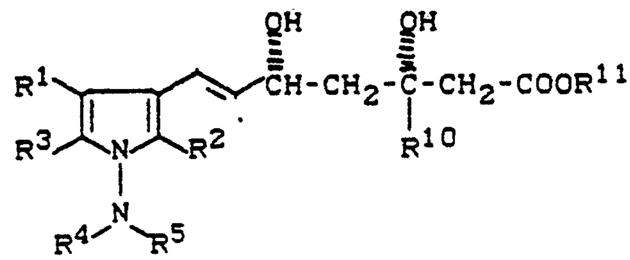

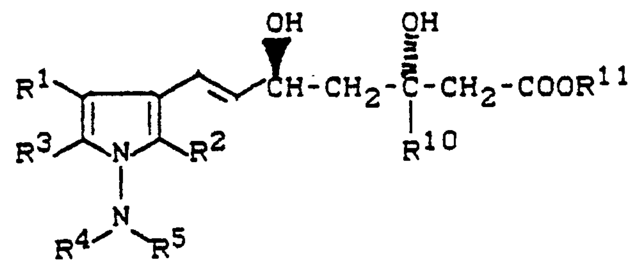

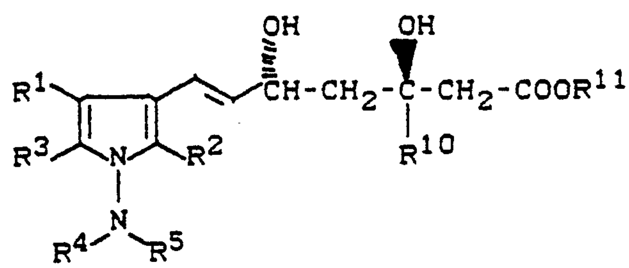

Ganz besonders bevorzugt sind die Verbindungen der allgemeinen Formel (Ia),
in welchen

$R^1$ - für Cyclopropyl, Methyl, Ethyl, Propyl, Isopropyl, Butyl, Isobutyl oder tert.-Butyl steht, das durch Fluor, Chlor, Hydroxy, Methoxy, Phenyl oder Phenoxy substituiert sein kann,

$R^2$ - für Phenyl steht, das bis zu 2-fach gleich oder verschieden durch Methyl, Ethyl, Propyl, Isopropyl, Butyl, Isobutyl, tert.-Butyl, Hydroxymethyl, Methoxy, Ethoxy, Propoxy, Phenoxy, Benzyloxy, Fluor, Chlor, Cyano, Trifluormethyl substituiert sein kann,

$R^3$ - für Wasserstoff steht,
- für Cyclopropyl steht,
- für Methyl, Ethyl, Propyl, Isopropyl steht, das substituiert sein kann durch Fluor, Hydroxy, Chlor, Cyano oder Trifluormethyl,
- für Phenyl steht, das substituiert sein kann durch Fluor, Chlor, Cyano, Hydroxy oder Trifluormethyl

$R^4$ und $R^5$ gleich oder verschieden sind und
- für Methyl, Ethyl, Propyl, Isopropyl, Butyl oder tert.-Butyl stehen oder
- für Phenyl stehen, das bis zu 2-fach gleich oder verschieden durch Methyl, Ethyl, Propyl, Isopropyl, Methoxy, Fluor, Chlor, Hydroxy, Trifluormethyl oder Methoxycarbonyl substituiert sein kann

oder

$R^4$ und $R^5$ gemeinsam mit dem N-Atom einen 5- bis 7-gliedrigen Heterocyclus bilden,
wobei dieser Heterocyclus durch $R^9$ substituiert sein kann,
wobei

$R^9$ - für Cyclopropyl oder
- für Methyl, Ethyl, tert.-Butyl oder
- für Pyridyl steht oder
- für Phenyl steht, das bis zu 2-fach gleich oder verschieden substituiert sein kann durch Methyl, Ethyl, Propyl, Fluor, Chlor, Cyano oder Trifluormethyl,
X - für eine Gruppe der Formel

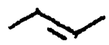 (E-Konfiguration) steht

und
A - für eine Gruppe der Formel

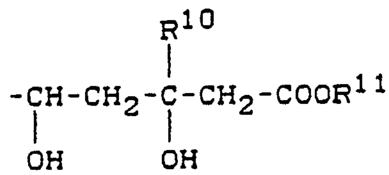 oder 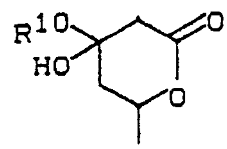 steht,

worin
$R^{10}$ - Wasserstoff bedeutet
und
$R^{11}$- Wasserstoff, Methyl oder Ethyl bedeutet oder ein Natrium- oder Kaliumkation bedeutet.

Außerdem wurde ein Verfahren zur Herstellung der substituierten Pyrrole der allgemeinen Formel (I)

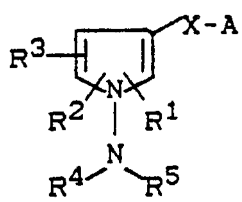 (I)

in welcher
$R^1$, $R^2$, $R^3$, $R^4$, $R^5$, X und A die obengenannte Bedeutung haben,
gefunden,
das dadurch gekennzeichnet ist, daß man
Ketone der allgemeinen Formel (VIII)

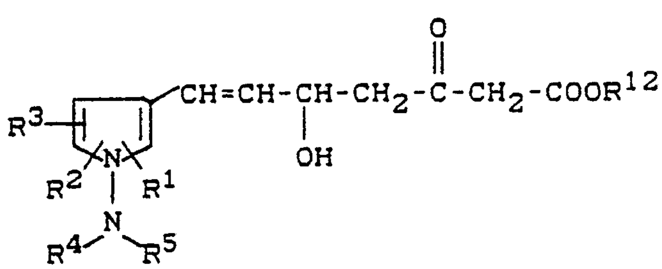

in welcher
$R^1$, $R^2$, $R^3$, $R^4$, $R^5$ die oben angegebene Bedeutung haben,
und
$R^{12}$ - für Alkyl steht,
reduziert,
im Fall der Herstellung der Säuren die Ester verseift,
im Fall der Herstellung der Lactone die Carbonsäuren cyclisiert,

im Fall der Herstellung der Salze entweder die Ester oder die Lactone verseift,
im Fall der Herstellung der Ethylenverbindungen (X = $-CH_2-CH_2-$) die Ethenverbindungen (X = -CH=CH-) nach üblichen Methoden hydriert,
und gegebenenfalls Isomeren trennt.

Das erfindungsgemäße Verfahren kann durch das folgendes Formelschema erläutert werden:

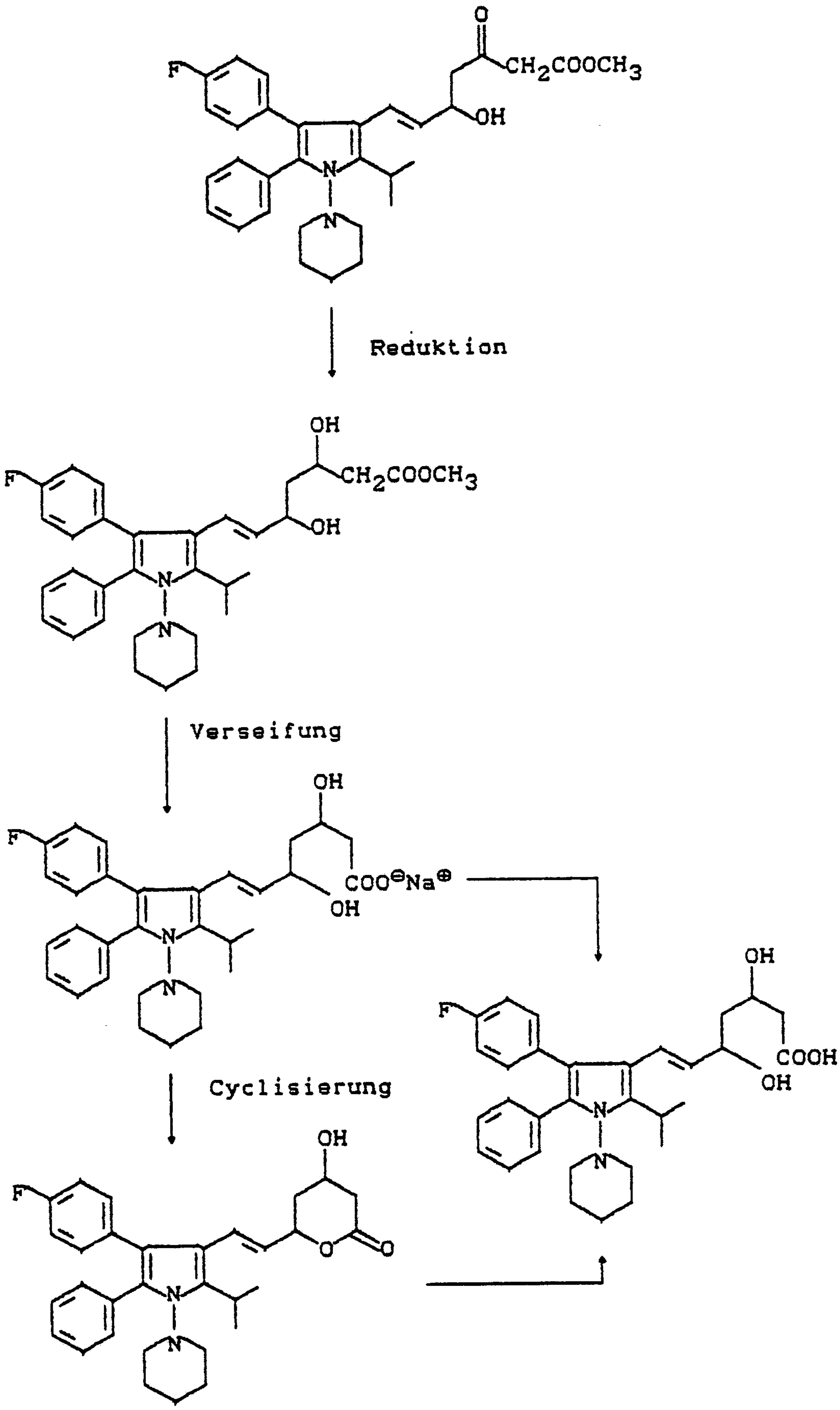
Reduktion
Verseifung
Cyclisierung

Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl-hydrido-borate, Natrium-trialkyl-hydrido-boranaten, Natrium-cyano-trihydrido-borat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluoi oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispiels weise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -90°C bis Raumtemperatur (+30°C), bevorzugt von -80°C bis 0°C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (I), in welchen X für eine Ethylengruppierung steht, kann die Reduktion der Ketone (III) unter solchen Bedingungen durchgeführt werden, unter denen sowohl die Carbonylgruppe als auch die Doppelbindung reduziert wird.

Darüber hinaus ist es auch möglich, die Reduktion der Carbonylgruppe und die Reduktion der Doppelbindung in zwei getrennten Schritten durchzuführen. Bevorzugt wird dabei die Doppelbindung nach Reduktion der Carbonylgruppe in Gegenwart eines Edelmetallkatalysators, wie beispielsweise Palladium oder Rhodium, hydriert (J. Med. Chem. 28, 347 (1985).

Die Carbonsäuren im Rahmen der allgemeinen Formel (I) entsprechen der Formel (Ic)

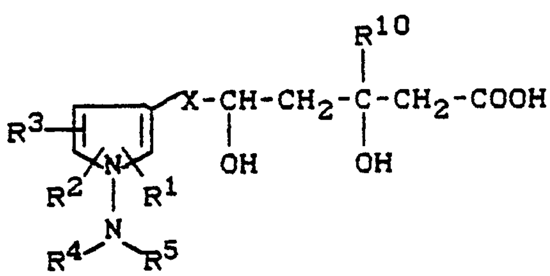

(Ic)

in welcher

$R^1$, $R^2$, $R^3$, $R^4$, $R^5$, $R^{10}$ und X die oben angegebene Bedeutung haben.

Die Carbonsäureester im Rahmen der allgemeinen Formel (I) entsprechen der Formel (Id)

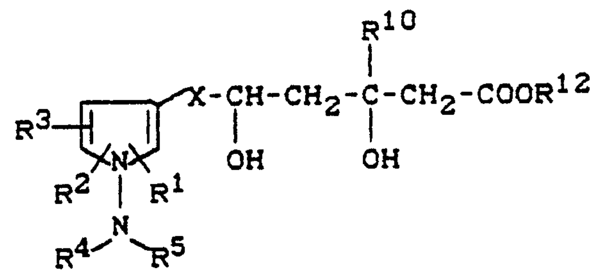

(Id)

in welcher
$R^1$, $R^2$, $R^3$, $R^4$, $R^5$, $R^{10}$ und X oben angegebene Bedeutung haben,
und
$R^{12}$ - für Alkyl steht.

Die Salze der erfindungsgemäßen Verbindungen im Rahmen der allgemeinen Formel (I) entsprechen der Formel (Ie)

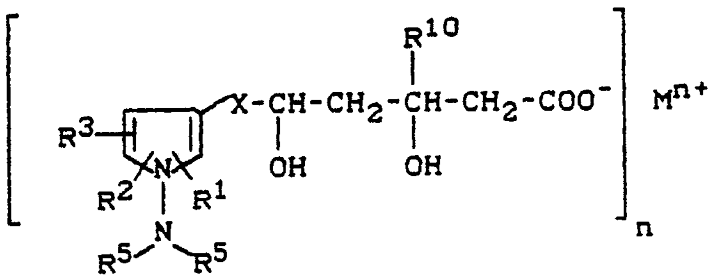

(Ie)

in welcher
$R^1$, $R^2$, $R^3$, $R^4$, $R^5$, $R^{10}$ und X die oben angegebene Bedeutung haben,
und
$M^{n+}$ für ein Kation steht.

Die Lactone im Rahmen der allgemeinen Formel (I) entsprechen der Formel (If)

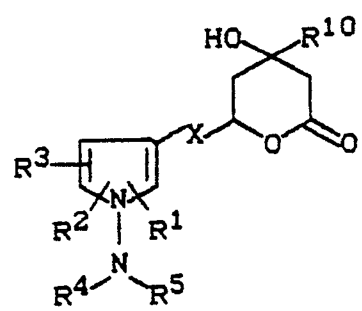

(If)

in welcher
$R^1$, $R^2$, $R^3$, $R^4$, $R^5$, $R^{10}$ und X die oben angegebene Bedeutung haben.

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säure in die freien Säuren der allgemeinen Formel (Ic) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ie) als Zwischenprodukte, die isoliert werden können. Die erfindungsgemäßen Säuren (Ic) erhält man durch Behandeln der Salze (Ie) mit üblichen anorganischen Säu ren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäuern. Die Säuren können dann in üblicher Weise isoliert werden.

Zur Herstellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40°C bis +200°C, bevorzugt von -25°C bis +50°C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cyclisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N′-Dicyclohexylcarbodiimid-Paratoluolsulfonat, N-Cyclohexyl-N′-[2-(N″-methylmorpholinium)ethyl]carbodiimid oder N-(3-Dimethylaminopropyl)-N′-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0°C bis +80°C, bevorzugt von +10°C bis +50°C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolt im allgemeinen nach üblichen Methoden wie sie beispielsweise von E.L. Eliel, Stereochemistry of Carbon Compounds, McGraw Hill, 1962 beschrieben wird. Bevorzugt wird hierbei die Trennung der Isomeren auf der Stufe der racemischen Lactone. Besonders bevorzugt wird hierbei die racemischen Mischung der trans-Lactone (VII) durch Behandeln entweder mit D-(+)-oder L-(-)-α-Methylbenzylamin nach üblichen Methoden in die diastereomeren Dihydroxyamide (Ig)

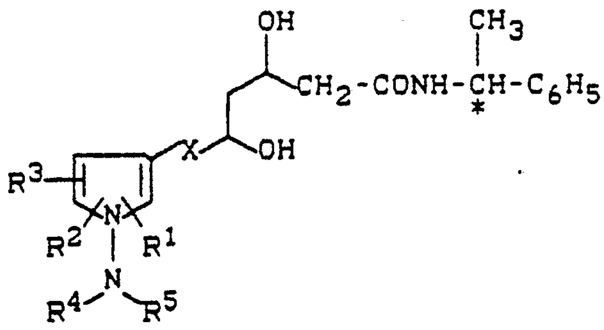

(Ig)

überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kalium hydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ic), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukt nach der oben beschriebenen Methode abhängig ist von der Konfiguration der Ausgangsstoffe.

Die Isomerentrennung soll im folgenden Schema beispielhaft erläutert werden:

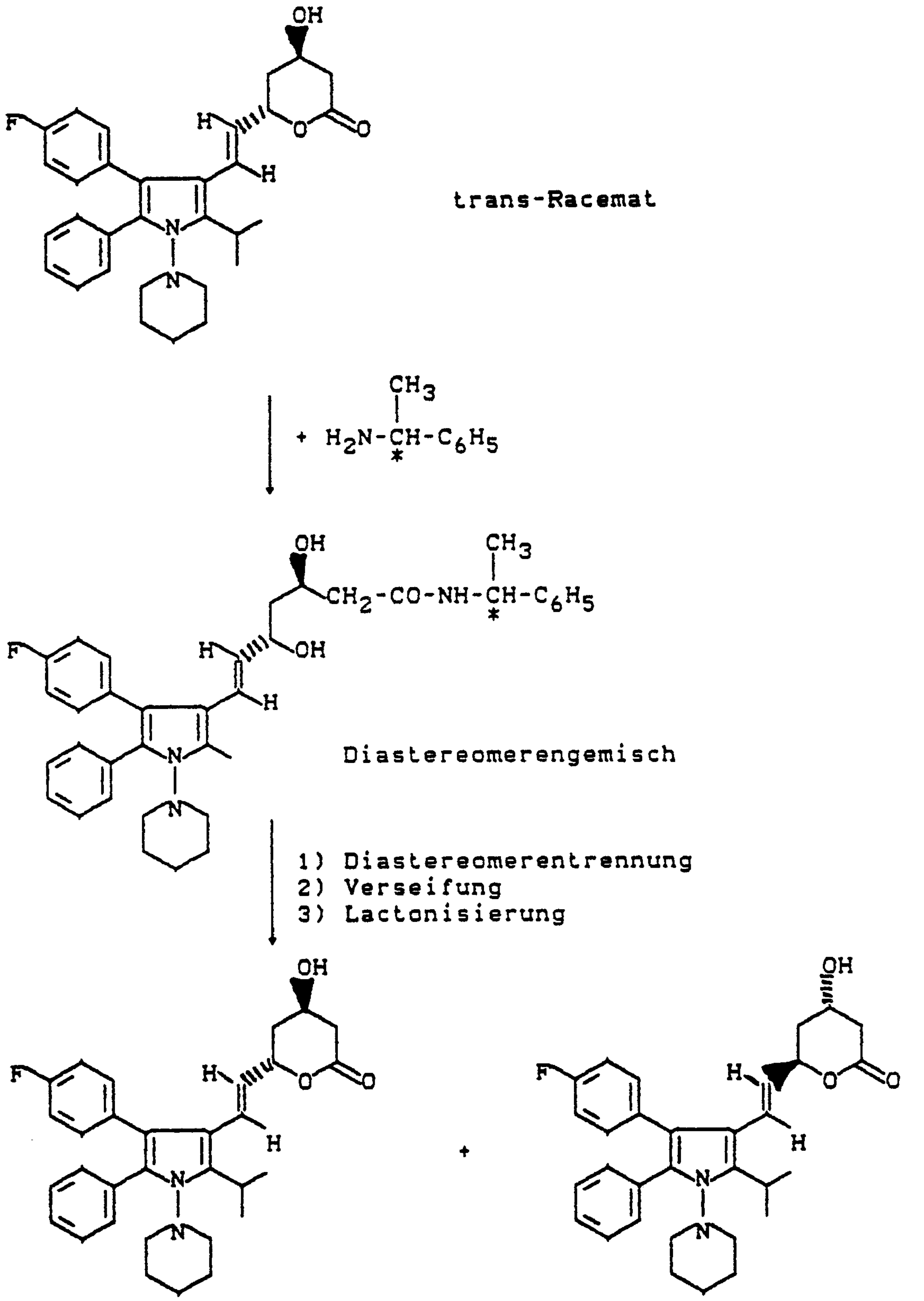


Die als Ausgangsstoffe eingesetzten Ketone (VIII) sind neu.

Es wurde ein Verfahren zur Herstellung der erfindungsgemäßen Ketone der allgemeinen Formel (VIII)

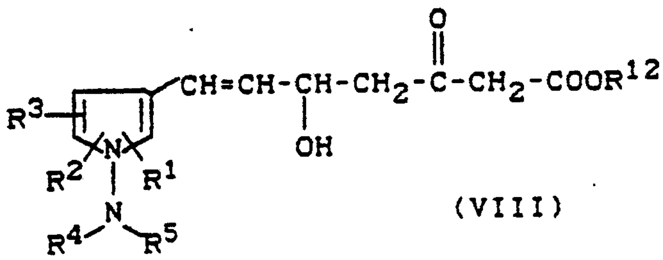

(VIII)

in welcher
$R^1$, $R^2$, $R^3$, $R^4$, $R^5$ und $R^{12}$ die obengenannte Bedeutung haben,
gefunden, das dadurch gekennzeichnet ist, daß man
Aldehyde der allgemeinen Formel (IX)

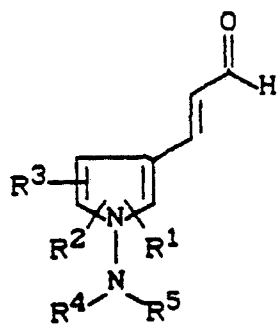

(IX)

in welcher
$R^1$, $R^2$, $R^3$, $R^4$ und $R^5$ die oben angegebene Bedeutung haben,
in inerten Lösemitteln mit Acetessigester der allgemeinen Formel (X)

$H_3C-\overset{O}{\overset{\|}{C}}-CH_2-COOR^{12}$ (X)

in welcher
$R^{12}$ die oben angegebene Bedeutung hat,
in Anwesenheit von Basen umsetzt.

Das erfindungsgemäße Verfahren kann beispielsweise durch folgendes Formelschema erläutert werden:

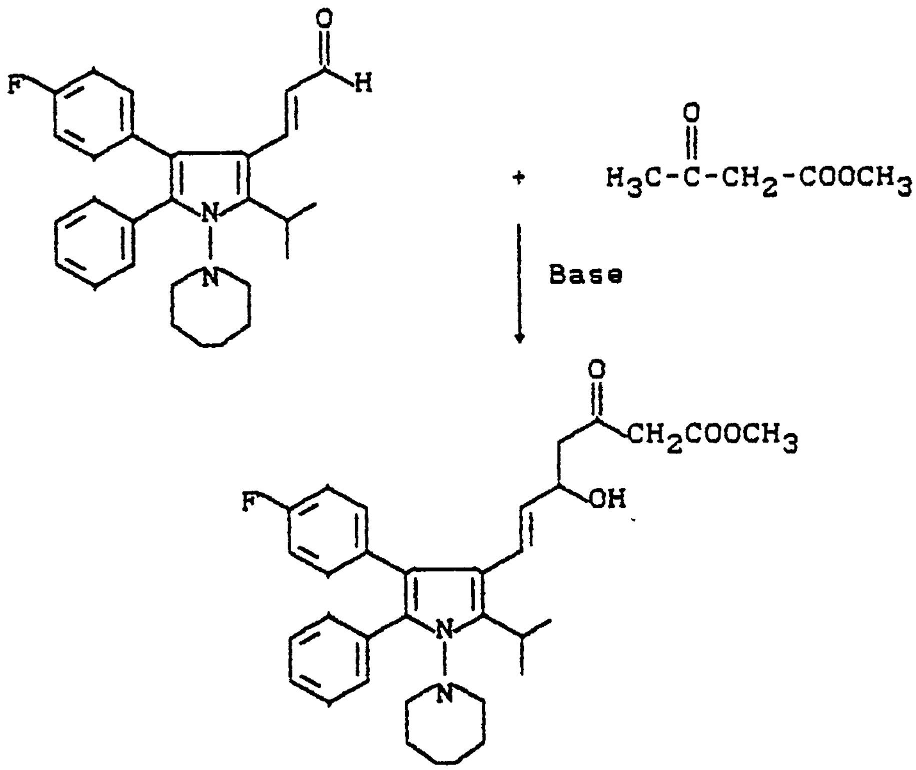


Als Basen kommen hierbei die üblichen stark basischen Verbindungen in Frage. Hierzu gehören bevorzugt lithiumorganische Verbindungen wie beispielsweise n-Butyllithium, sec.Butyllithium, tert.Butyllithium oder Phenyllithium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden n-Butyllithium oder Natriumhydrid oder deren Gemisch eingesetzt.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80° C bis +50° C, bevorzugt von -20° C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt:
Acetessigsäuremethylester, Acetessigsäureethylester, Acetessigsäurepropylester, Acetessigsäureisopropylester.

Die als Ausgangsstoffe eingesetzten Aldehyde der allgemeinen Formel (IX) sind neu.

Es wurde außerdem ein Verfahren zur Herstellung der Aldehyde der allgemeinen Formel (IX)

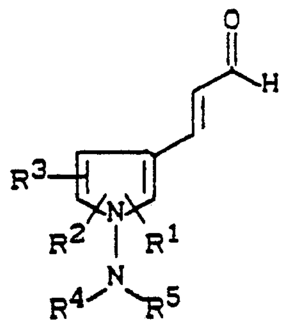

(IX)

in welcher
$R^1$, $R^2$, $R^3$, $R^4$, und $R^5$ die oben angebene Bedeutung haben,
gefunden, das dadurch gekennzeichnet ist, daß man
Pyrrole der allgemeinen Formel (XI)

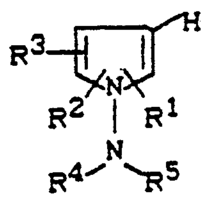

(XI)

in welcher
$R^1$, $R^2$, $R^3$, $R^4$ und $R^5$ die oben angegebene Bedeutung haben,
in inerten Lösemitteln in Anwesenheit von Säurehalogeniden mit N,N-Dialkylaminoacrolein der Formel (XII)

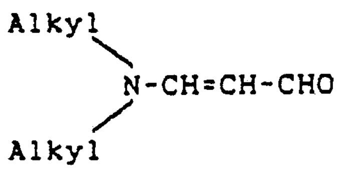

(XII)

worin
Alkyl für einen geradkettigen oder verzweigten Kohlenwasserstoffrest ($C_1$ bis $C_4$) steht,
umsetzt.

Das erfindungsgemäße Verfahren kann beispielsweise durch folgendes Formelschema verdeutlicht werden:

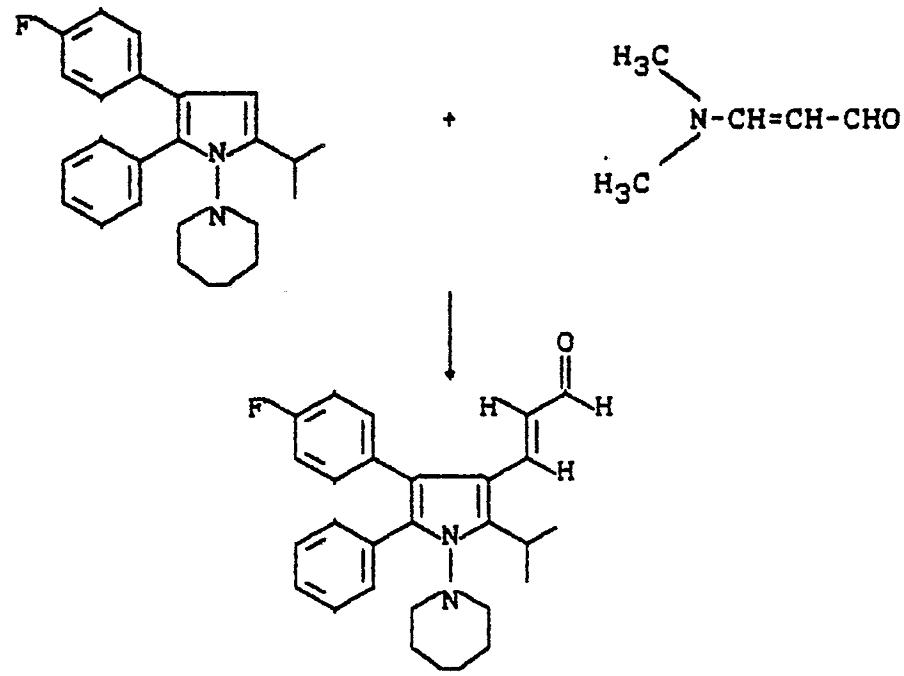

Als Lösemittel eignen sich hierbei die üblichen organischen Lösemittel, die unter den Reaktionsbedingungen stabil sind. Hierzu gehören bevorzugt Kohlenwasserstoffe wie Benzol, Toluol, Xylol, Hexan, Erdölfraktionen, halogenierte Kohlenwasserstoffe, wie Chlorbenzol oder Dichlorbenzol, oder Ether wie Diethylether, Dioxan oder Tetrahydrofuran, oder Chlorkohlenwasserstoffe wie Methylenchlorid, Chloroform oder Tetrachlorkohlenstoff, oder Acetonitril. Ebenso ist es möglich, Gemische der genannten Lösemittel einzusetzen. Besonders bevorzugt wird wasserfreies Acetonitril oder Chloroform verwendet.

Als Hilfsstoffe werden im allgemeinen Säurechloride verwendet. Bevorzugt wird Phosphoroxychlorid oder Phosgen, besonders bevorzugt Phosphoroxychlorid eingesetzt.

Die Reaktion wird in einem Temperaturbereich von -20°C bis +150°C, bevorzugt von 0°C bis +100°C durchgeführt.

Das Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung des Verfahrens wird das Dimethylaminoacrolein im allgemeinen in einer Menge von 2 bis 6, bevorzugt von 3 bis 4 mol, bezogen auf 1 mol des Pyrrols eingesetzt.

Die als Ausgangsstoffe eingesetzten Pyrrole der allgemeinen Formel (XI) sind bekannt oder können nach bekannten Methoden hergestellt werden [A. Glossauer "Die Chemie der Pyrrole", Springer Verlag Berlin, 1974].

Die erfindungsgemäßen N-substituierten N-Amino-pyrrole besitzen wertvolle pharmakologische Eigenschaften und können als Wirkstoffe in Arzneimitteln eingesetzt werden. Insbesondere sind sie Inhibitoren der 3-Hydroxy-3-methyl-glutarylCoenzym A (HGM-CoA) Reduktase und Inhibitoren der Cholesterolbiosynthese. Sie können zur Behandlung von Hyperlipoproteinämie, Lipoproteinämie oder Arteriosklerose eingesetzt werden. Die erfindungsgemäßen Wirkstoffe bewirken außerdem eine Senkung des Cholesteringehaltes im Blut.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin),

Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art der Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Ausgangsverbindungen und Herstellungsbeispiele

Beispiel 1

1-(4-Fluorphenyl)-4-methyl-pent-1-en-3-on

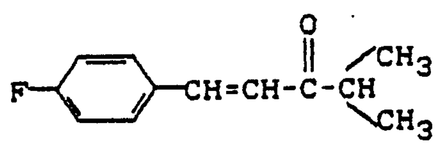

Zu 198,4 g (1,6 mol) frisch destilliertem 4-Fluorbenzaldehyd und 137,6 g (1,6 mol) Methylisopropylketon in 300 ml Methanol tropft man 75 ml 15%ige Kalilauge und rührt über Nacht bei Raumtemperatur. Dann wird mit 10 ml Essigsäure neutralisiert, 1 l Wasser zugesetzt und mit zwei 500 ml Portionen Ether extrahiert. Die vereinigten organischen Phasen werden mit 500 ml gesättigter Natriumchlorid-Lösung gewaschen und über Natriumsulfat getrocknet. Nach Abziehen des Lösemittels destilliert man im Hochvakuum.

Ausbeute: 198,6 g (65% der Theorie) gelbliches Öl

Kp.: 103 °C (0.3 mbar)

$^1$H-NMR ($CDCl_3$): $\delta$ = 1,2 (d, 6H, $CH_3$); 2,9 (sept, 1H, CH-$(CH_3)_2$); 6,8 (d, 1H, Olefin-H); 7,1 (m, 2H, Aromaten-H); 7,6 (m, 3H, Aromaten-H + Olefin-H).

Beispiel 2

2-(4-Fluorphenyl)-5-methyl-1-phenyl-hexan-1,4-dion

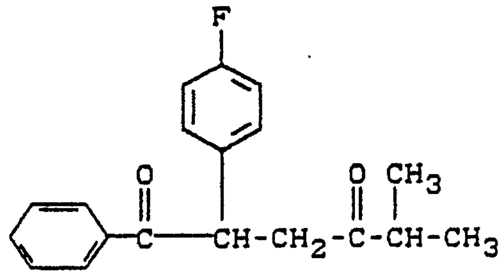

Zu einer Lösung von 5,88 g (0,12 mol) Natriumcyanid in 300 ml Dimethylformamid, tropft man bei 35 °C eine Lösung von 63,6 g (0,6 mol) frisch destilliertem Benzaldehyd in 300 ml Dimethylformamid innerhalb von 30 min und rührt weitere 5 min bei dieser Temperatur. Dann werden innerhalb von 1,5 h 86,5 g (0,45 mol) 1-(4-Fluorphenyl)-4-methyl-pent-1-en-3-on (Beispiel 1) in 500 ml Dimethylformamid zugetropft und 1 h nachgerührt, wobei die Temperatur stets bei 35 °C gehalten wird.
Nach Versetzen mit 1 l Wasser wird viermal mit je 400 ml Chloroform extrahiert, die vereinigten organischen Phasen mit 1 l gesättigter Natriumhydrogencarbonat-Lösung und 1 l Wasser gewaschen und über Natriumsulfat getrocknet. Nach dem Einengen im Vakuum destilliert man unter vermindertem Druck bis zuletzt eine Fraktion bei 138 - 142 °C (0,9 mbar) übergeht. Der Destillationsrückstand (132 g) wird nun in zwei Portionen an einer Säule (1,5 Kg Kieselgel 230 - 400 mesh, ⌀ 9 cm), mit Petrolether / Essigester (10 : 1) chromatographiert. Das Produkt wird nach 4 - 7 l Elutionsmittel erhalten. Nach Abziehen des Lösemittels bleiben 90,0 g (67% der Theorie), farbloses Öl.
$^1$H-NMR ($CDCl_3$): δ = 1,08 (d, 3H, $CH_3$); 1,12 (d, 3H, $CH_3$); 2,65 (sept, 1H, <u>CH</u>-$(CH_3)_2$); 2,7 (dd, 1H, -CO-$CH_2$-CH); 3,6 (dd, 1H, -CO-$CH_2$-CH); 5,12 (dd, 1H, H-C-$C_6H_4$-F); 6,95 (m, 2H, Aromaten-H); 7,23 (m, 2H, Aromaten-H); 7,4 (m, 3H, Aromaten-H); 7,95 (m, 2H, Aromaten-H).

Beispiel 3

3-(4-Fluorphenyl)-5-isopropyl-2-phenyl-1-(1-pyrrolidinyl)-pyrrol

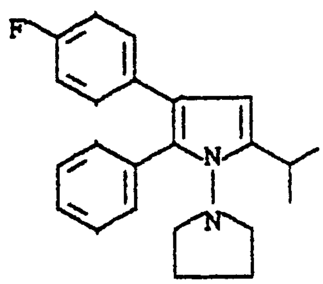

15 g (50 mmol) der Verbindung aus Beispiel 2 und 20 g (164 mmol) N-Aminopyrrolidin-Hydrochlorid werden in 150 ml Toluol p.A. und 30 ml Dimethylformamid mit 20 g Molekularsieb 3Å versetzt und 48 h am Rückfluß gekocht. Die Mischung wird abgekühlt, abfiltriert und gut mit Toluol nachgewaschen. Die Toluollösungen werden vereinigt und 3 x mit 1 N Salzsäure extrahiert. Die orga nische Phase wird anschließend mit gesättigter Natriumhydrogencarbonat-Lösung gewaschen, über Magnesiumsulfat getrocknet und im Vakuum eingeengt. Der Rückstand wird mit wenig Ethanol verrührt, abgekühlt und abgesaugt. Nach Trocknen im Exsikkator erhält man 2,3 g Substanz,
Ausbeute: 12,8 % der Theorie.
$^1$H-NMR ($CDCl_3$): δ = 1,31 (d, 6H); 1,5-1,8 (m, 4H); 3,05 (m, 1H); 3,23 (m, 4H); 6,03 (s, 1H); 6,8 (m, 2H); 7,07 (m, 2H); 7,35 (m, 5H).

Beispiel 4

(E)-3-[3-(4-Fluorphenyl)-5-isopropyl-2-phenyl-1-(1-pyrrolidinyl)-pyrrol-4-yl]-prop-2-enal

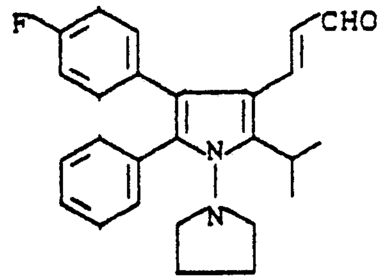

Unter Stickstoff werden zu 1,8 ml (19,2 mmol) frisch destilliertem Phosphoroxidchlorid in 20 ml Acetonitril p.A. bei -5 °C 1,9 g (17,9 mmol) 90 %iges 3-Dimethylamino-acrolein in 30 ml Acetonitril p.A. getropft. Man rührt 10 Minuten nach und tropft bei derselben Temperatur 2,3 g (6,4 mmol) der Verbindung aus Beispiel 3 gelöst in 20 ml Acetonitril p.A. und 30 ml Tetrahydrofuran p.A. zu. Anschließend wird über Nacht am Rückfluß gekocht und auf Raumtemperatur abgekühlt. Der Ansatz wird auf ein Gemisch von 4,7 g Natriumhydroxid in 300 ml Toluol/Wasser 1:1 gegeben, so daß die Temperatur 10 °C nicht übersteigt. Man rührt noch 10 Minuten nach, trennt die organische Phase ab und extrahiert die wäßrige Phase zweimal mit Essigester. Die organischen Phasen werden vereinigt, mit gesättigter Natriumchlorid-Lösung gewaschen und über Magnesiumsulfat getrocknet. Nach Einengen im Vakuum wird der Rückstand mit Essigester/Petrolether 1:1 verrührt, abgesaugt und im Exsikkator getrocknet.
Ausbeute: 2 g (71,4 % der Theorie).
$^1$H-NMR ($CDCl_3$): $\delta$ = 1,48 (d, 6H); 1,72 (m, 4H); 3,23 (m, 4H); 3,5 (m, 1H); 5,75 (dd, 1H); 6,85 (m, 2H); 7,05 (m, 2H); 7,25 (m, 5H); 7,55 (d, 1H); 9,38 (d, 1H).

Beispiel 5

Methyl-(E)-7-[3-(4-fluorphenyl)-5-isopropyl-2-phenyl-1-(1-pyrrolidinyl)-pyrrol-4-yl]-5-hydroxy-3-oxo-hept-6-enoat

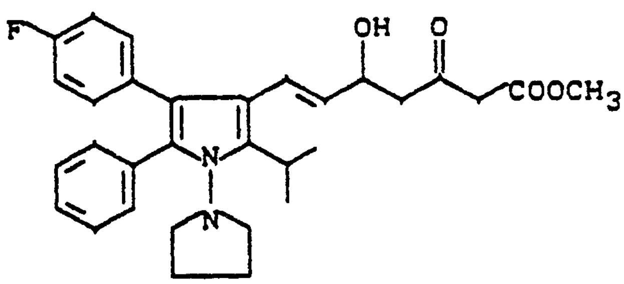

Unter Stickstoff tropft man zu einer Suspension von 360 mg (12 mmol) 80 %igem Natriumhydrid in 30 ml trockenem Tetrahydrofuran bei -5 °C 1,1 ml (10 mmol) Acetessigsäuremethylester in 5 ml trockenem Tetrahydrofuran. Nach 15 Minuten werden bei derselben Temperatur 6,2 ml (10 mmol) 15 %iges Butyllithium in n-Hexan zugetropft und 15 Minuten nachgerührt. Anschließend werden 2 g (5 mmol) der Verbindung aus Beispiel 4, gelöst in 20 ml trockenem Tetrahydrofuran, zugetropft und 30 Minuten bei -5 °C nachgerührt. Die Reaktionslösung wird vorsichtig mit 3,3 ml 50 %iger Essigsäure versetzt, mit 100 ml Wasser verdünnt und die Mischung dreimal mit je 100 ml Ether extrahiert. Die vereinigten organischen Phasen werden zweimal mit gesättigter Natriumhydrogencarbonat-Lösung und einmal mit gesättigter Natriumchlorid-Lösung gewaschen, über Magnesiumsulfat getrocknet und im Vakuum eingeengt. Der Rückstand wird an einer Säule (Kieselgel 70-230 mesh, mit Essigester/Petrolether 4:6) chromatographiert.
Ausbeute: 1 g (40 % der Theorie)
$^1$H-NMR ($CDCl_3$): $\delta$ = 1,42 (d, 6H); 1,50 (m, 2H); 1,72 (m, 2H); 2,62 (m, 2H); 3,22 (m, 4H); 3,32 (m, 1H);

3,46 (s, 2H); 3,73 (s, 3H); 4,55 (m, 1H); 5,12 (dd, 1H); 6,62 (d, 1H); 6,82 (m, 2H); 7,03 (m, 2H); 7,23 (m, 5H).

Beispiel 6

Methyl-erythro-(E)-7-[3-(4-fluorphenyl)-5-isopropyl-2-phenyl-1-(1-pyrrolidinyl)-pyrrol-4-yl]-3,5-dihydroxy-hept-6-enoat

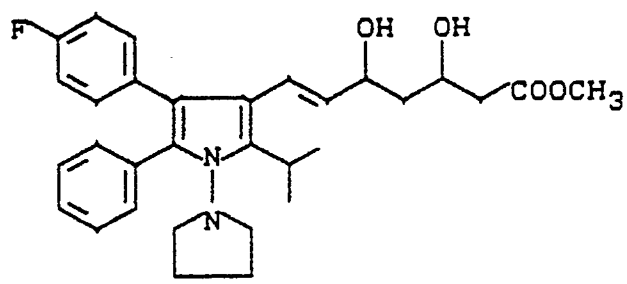

Zu einer Lösung von 1 g (1,93 mmol) der Verbindung aus Beispiel 5 in 30 ml trockenem Tetrahydrofuran gibt man bei Raumtemperatur 2,4 ml (2,4 mmol) einer 1-molaren Triethylboran-Lösung in Tetrahydrofuran, leitet während 5 Minuten Luft durch die Lösung und kühlt auf -30 °C Innentemperatur ab. Es werden 91 mg (2,4 mmol) Natriumborhydrid und langsam 1,8 ml Methanol zugegeben, 30 Minuten bei -30 °C gerührt und dann mit einem Gemisch aus 8,5 ml 30 %igem Wasserstoffperoxid und 17 ml Wasser versetzt. Die Temperatur läßt man dabei bis 0 °C ansteigen und rührt noch 30 Minuten nach. Die Mischung wird dreimal mit je 50 ml Essigester extrahiert, die vereinigten organischen Phasen je einmal mit gesättigter Natriumhydrogencarbonat-Lösung und gesättigter Natriumchlorid-Lösung gewaschen, über Magnesiumsulfat getrocknet und im Vakuum eingeengt. Der Rückstand wird an einer Säule (Kieselgel 230-400 mesh, mit Essigester/Petrolether 1:1) chromatographiert.
Ausbeute: 400 mg (40 % der Theorie).
$^1$H-NMR ($CDCl_3$): δ = 1,42 (d, 6H); 1,40-1,80 (m, 6H); 2,47 (m, 2H); 3,22 (m, 4H); 3,32 (m, 1H); 3,70 (2, 3H); 4,22 (m, 1H); 4,37 (m, 1H); 5,17 (dd, 1H); 6,58 (d, 1H); 6,80 (m, 2H); 7,03 (m, 2H); 7,23 (m, 5H).

Beispiel 7

3-(4-Fluorphenyl)-5-isopropyl-2-phenyl-1-piperidinopyrrol

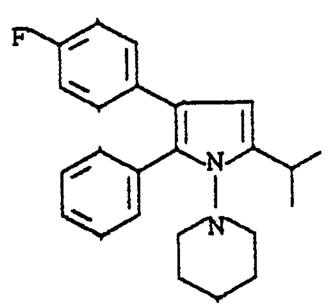

22,5 g (75 mmol) der Verbindung aus Beispiel 2 und 25 g (250 mmol) N-Aminopiperidin in 200 ml Toluol p.A. und 30 ml Dimethylformamid werden mit 7,5 ml konz. Salzsäure vesetzt und 48 h am Wasserabscheider zum Rückfluß erhitzt. Die Mischung wird abgekühlt, mit 200 ml Essigester verdünnt, dreimal mit 1 N Salzsäure und zweimal mit gesättigter Natriumhydrogencarbonat-Lösung extrahiert. Die organische Phase wird über Magnesiumsulfat getrocknet, im Vakuum eingeengt und der Rückstand an

einer Säule (Kieselgel 70-230 mesh, mit Essigester/Petrolether 95:5) chromatographiert.
Ausbeute: 7,2 g (26,5 % der Theorie).
$^1$H-NMR ($CDCl_3$): $\delta$ = 1,0 (m, 1H); 1,26 (d, 6H); 1,35- 1,6 (m, 5H); 2,88 (m, 2H); 3,1 (m, 3H); 5,95 (s, 1H); 6,75 (m, 2H); 7,0 (m, 2H); 7,31 (m, 5H).

Beispiel 8

(E)-3-[3-(4-Fluorphenyl)-5-isopropyl-2-phenyl-1-piperidino-pyrrol-4-yl]-prop-2-enal

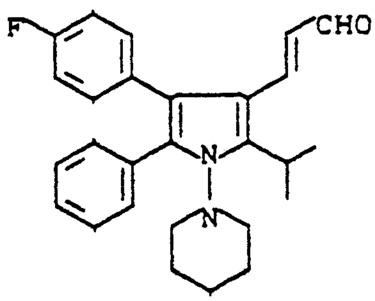

7,2 g (20 mmol) der Verbindung aus Beispiel 7 werden analog Beispiel 4 umgesetzt.
Ausbeute: 7,2 g (86,7 % der Theorie).
$^1$H-NMR ($CDCl_3$): $\delta$ = 0,93 (m, 1H); 1,48 (d, 6H); 1,5-1,7 (m, 5H); 2,92 (m, 2H); 3,15 (m, 2H); 3,55 (m, 1H); 5,75 (dd, 1H); 6,83 (m, 2H); 7,0 (m, 2H); 7,25 (m, 5H); 7,52 (d, 1H); 9,37 (d, 1H).

Beispiel 9

Methyl-(E)-7-[3-(4-fluorphenyl-5-isopropyl-2-phenyl-1-piperidino-pyrrol-4-yl]-5-hydroxy-3-oxo-hept-6-enoat

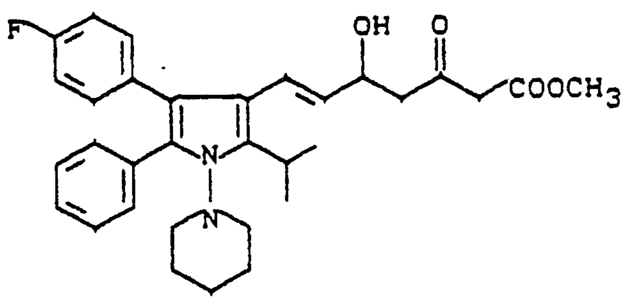

7,2 g (17,3 mmol) der Verbindung aus Beispiel 8 werden analog Beispiel 5 umgesetzt.
Ausbeute: 6,2 g (67,4 % der Theorie).
$^1$H-NMR ($CDCl_3$): $\delta$ = 1,40 (d, 6H); 0,90-1,60 (m, 6H); 2,62 (m, 2H); 2,90 (m, 2H); 3,12 (m, 2H); 3,38 (m, 1H); 3,47 (s, 2H); 3,73 (s, 3H); 4,53 (m, 1H); 5,15 (dd, 1H); 6,54 (d, 1H); 6,80 (m, 2H); 7,01 (m, 2H); 7,25 (m, 5H).

Beispiel 10

Methyl-erythro-(E)-7-[3-(4-fluorphenyl)-5-isopropyl-2-phenyl-1-piperidino-pyrrol-4-yl]-3,5-dihydroxy-hept-6-

enoat

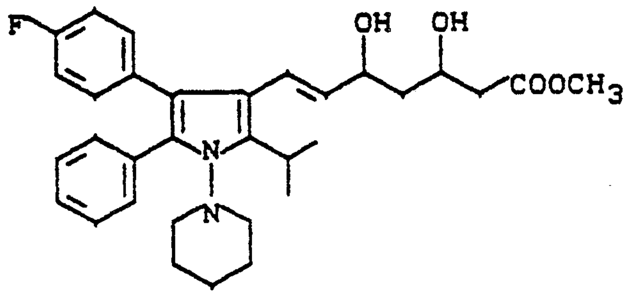

6,2 g (11,7 mmol) der Verbindung aus Beispiel 9 werden analog Beispiel 6 umgesetzt.
Ausbeute: 4,3 g (69,4 % der Theorie).
$^1$H-NMR ($CDCl_3$): $\delta$ = 1,42 (d, 6H); 0,90-1,70 (m, 8H); 2,47 (m, 2H); 2,92 (m, 2H); 3,13 (m, 2H); 3,41 (m, 1H); 3,72 (s, 3H); 4,20 (m, 1H); 4,36 (m, 1H); 5,19 (dd, 1H); 6,52 (d, 1H); 6,78 (m, 2H); 7,02 (m, 2H); 7,23 (m, 5H).

Beispiel 11

1-(4-Fluor-3-phenoxyphenyl)-4-methyl-pent-1-en-3-on

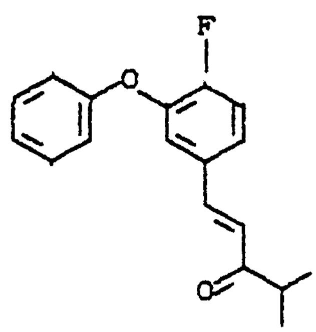

64,8 g (0,3 mol) 3-Phenoxy-4-fluorbenzaldehyd und 51,6 g (0,6 mol) Methylisopropylketon in 90 ml Methanol werden mit 14,1 ml 15 %iger wäßriger Kaliumhyroxid-Lösung versetzt und über Nacht bei Raumtemperatur gerührt. Die Mischung wird mit 1,95 ml Eisessig neutralisiert, mit 300 ml Wasser verdünnt und mehrmals mit Ether extrahiert. Die organische Phase wird über Magnesiumsulfat getrocknet, im Vakuum eingeengt und der Rückstand an einer Säule (Kieselgel 70-230 mesh, mit Essigester/Petrolether 1:9) chromatographiert.
Ausbeute: 41,6 g (48,8 % der Theorie).

Beispiel 12

2-(4-Fluor-3-phenoxyphenyl)-5-methyl-1-phenyl-hexan-1,4-dion

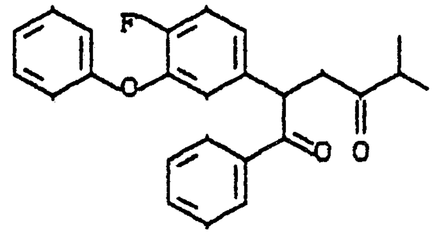

12,5 g (44 mmol) der Verbindung aus Beispiel 11 werden analog Beispiel 2 umgesetzt.
Ausbeute: 9,5 g (55,5 % der Theorie).

Beispiel 13

3-(4-Fluor-3-phenoxyphenyl)-5-isopropyl-2-phenyl-1-piperidino-pyrrol

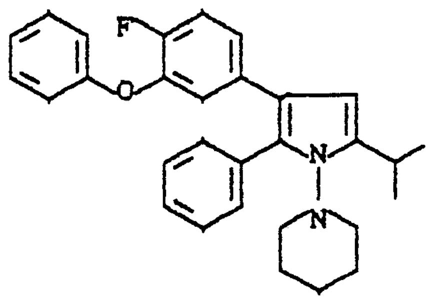

18,45 g (47,4 mmol) der Verbindung aus Beispiel 12 werden analog Beispiel 7 umgesetzt.
Ausbeute: 5,1 g (23,7 % der Theorie).
$^1$H-NMR ($CDCl_3$): $\delta$ = 1,0 (m, 1H); 1,28 (d, 6H); 1,4-1,6 (m, 5H); 2,89 (m, 2H); 3,1 (m, 3H); 5,95 (s, 1H); 6,2-7,35 (m, 13H).

Beispiel 14

(E)-3-[3-(4-Fluor-3-phenoxyphenyl)-5-isopropyl-2-phenyl-1-piperidino-pyrrol-4-yl]-prop-2-enal

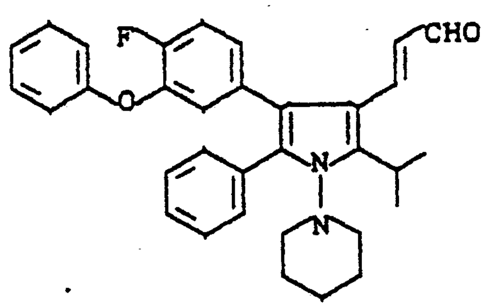

5,1 g (11,2 mmol) der Verbindung aus Beispiel 13 werden analog Beispiel 4 umgesetzt.
Ausbeute: 5 g (87,7 % der Theorie).
$^1$H-NMR ($CDCl_3$): $\delta$ = 0,9 (m, 1H); 1,45 (m, 6H); 1,52 (m, 5H); 2,9 (m, 2H); 3,1 (m, 2H); 3,5 (m, 1H); 5,81

(dd, 1H); 6,7-7,35 (m, 13H); 7,5 (d, 1H); 9,4 (d, 1H).

Beispiel 15

Methyl-(E)-7-[3-(4-fluor-3-phenoxyphenyl)-5-isopropyl-2-phenyl-1-piperidino-pyrrol-4-yl]-5-hydroxy-3-oxo-hept-6-enoat

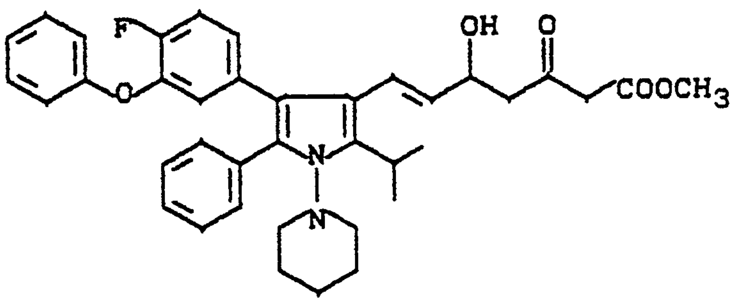

5 g (9,8 mmol) der Verbindung aus Beispiel 14 werden analog Beispiel 5 umgesetzt.
Ausbeute: 3,5 g (57,3 % der Theorie).
$^1$H-NMR ($CDCl_3$): $\delta$ = 1,37 (d, 6H); 0,90-1,60 (m, 6H); 2,62 (m, 2H); 2,87 (m, 2H); 3,08 (m, 2H); 3,37 (m, 1H); 3,45 (s, 2H); 3,73 (s, 3H); 4,54 (m, 1H); 5,22 (dd, 1H); 6,52 (d, 1H); 6,71 (m, 2H); 6,78 (m, 1H); 6,92 (m, 1H); 7,02 (m, 1H); 7,23 (m, 8H).

Beispiel 16

Methyl-erythro-(E)-7-[3-(4-fluor-3-phenoxyphenyl)-5-isopropyl-2-phenyl-1-piperidino-pyrrol-4-yl)-3,5-dihydroxy-hept-6-enoat

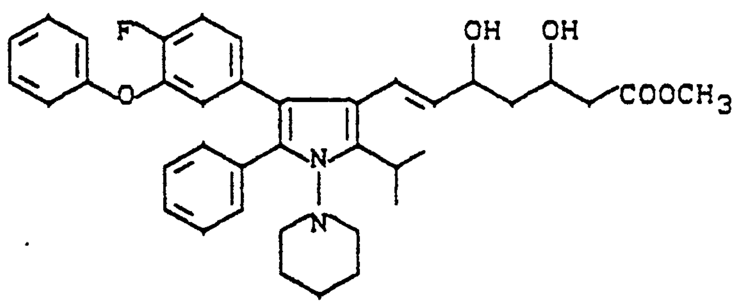

3,5 g (5,6 mmol) der Verbindung aus Beispiel 1 werden analog Beispiel 6 umgesetzt.
Ausbeute: 2,3 g (65,5 % der Theorie).
$^1$H-NMR ($CDCl_3$): $\delta$ = 1,37 (d, 6H); 0,9-1,7 (m, 8H); 2,47 (m, 2H); 2,90 (m, 2H); 3,12 (m, 2H); 3,38 (m, 1H); 3,72 (s, 3H); 4,22 (m, 1H); 4,37 (m, 1H); 5,26 (dd, 1H); 6,51 (d, 1H); 6,65-7,10 (m, 5H); 7,25 (m, 8H).

Beispiel 17

3-(4-Fluor-3-phenoxyphenyl)-6-methyl-heptan-2,5-dion

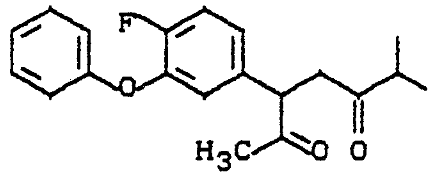

10 g (35,2 mmol) der Verbindung aus Beispiel 11 in 30 ml Acetonitril p.A. werden bei 0 °C mit 3 ml (42,2 mmol) Nitroethan versetzt. Anschließend werden bei derselben Temperatur 2,77 g (17,6 mmol) 1,8-Diazabicyclo(5,4,0)undec-7-en gelöst in 10 ml Acetonitril p.A., zugetropft und 1 Stunde bei Raumtemperatur nachgerührt. Der Ansatz wird mit 35 ml 0,5 N Salzsäure versetzt, mehrmals mit Dichlormethan extrahiert und die organische Phase über Magnesiumsulfat getrocknet. Nach Einengen im Vakuum erhält man einen öligen Rückstand, der in 35 ml Ethanol aufgenommen und mit einer Lösung von 1,7 g (42,2 mmol) Natriumhydroxid in 21 ml Wasser versetzt wird. Diese Mischung tropft man bei 0 °C zu 4,68 ml (88 mmol) konz. Schwefelsäure in 35 ml Wasser und läßt 30 Minuten bei Raumtemperatur rühren. Man verdünnt mit Wasser und extrahiert mehrmals mit Ether. Die organische Phase wird je zweimal mit 1 N Salzsäure und gesättigter Natriumhydrogencarbonat-Lösung gewaschen, über Magnesiumsulfat getrocknet und im Vakuum eingeengt. Nach Chromatographie an einer Säule (Kieselgel 70-230 mesh, mit Essigester/Petrolether 2:8) erhält man 4,8 g Substanz.

Ausbeute: 41,5 % der Theorie.

$^1$H-NMR ($CDCl_3$): $\delta$ = 1,05 (d, 3H); 1,1 (d, 3H); 2,15 (s, 3H); 2,58 (m, 2H); 3,35 (m, 1H); 4,15 (m, 1H); 6,85-7,38 (m, 8H).

## Beispiel 18

3-(4-Fluor-3-phenoxyphenyl)-5-isopropyl-2-methyl-1-piperidino-pyrrol

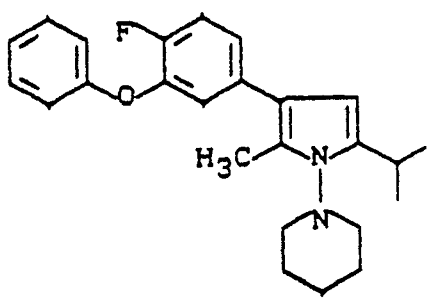

11,2 g (34 mmol) der Verbindung aus Beispiel 17 werden analog Beispiel 7 umgesetzt.

Ausbeute: 4,2 g (31,5 % der Theorie).

$^1$H-NMR ($CDCl_3$): $\delta$ = 1,22 (d, 6H); 1,30 (m, 1H); 1,72 (m, 5H); 2,38 (s, 3H); 3,06 (m, 1H); 3,15 (m, 2H); 3,30 (m, 2H); 5,77 (s, 1H); 7,0-7,40 (m, 8H).

## Beispiel 19

(E)-3-[3-(4-Fluor-4-phenoxyphenyl)-5-isopropyl-2-methyl-1-piperidino-pyrrol-4-yl]-prop-2-enal

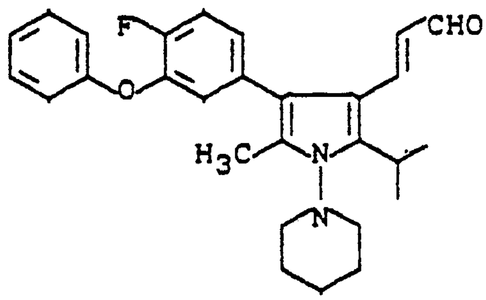

4,15 g (10,6 mmol) der Verbindung aus Beispiel 18 werden analog Beispiel 4 umgesetzt.
Ausbeute: 2 g (42,6 % der Theorie).
$^1$H-NMR ($CDCl_3$): $\delta$ = 1,30 (m, 1H); 1,38 (d, 6H); 1,73 (m, 5H); 2,18 (s, 3H); 3,13 (m, 2H); 3,32 (m, 2H); 3,43 (m, 1H); 5,73 (dd, 1H); 6,70-7,45 (m, 8H); 7,40 (d, 1H); 9,33 (d, 1H).

Beispiel 20

Methyl-(E)-7-[3-(4-fluor-3-phenoxyphenyl)-5-isopropyl-2-methyl-1-piperidino-pyrrol-4-yl]-5-hydroxy-3-oxo-hept-6-enoat

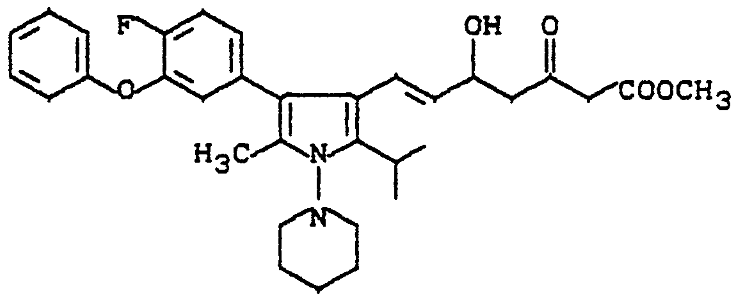

2 g (4,5 mmol) der Verbindung aus Beispiel 19 werden analog Beispiel 5 umgesetzt.
Ausbeute: 1,7 g (67,2 % der Theorie).
$^1$H-NMR ($CDCl_3$): $\delta$ = 1,32 (d, 6H); 1,20-1,80 (m, 6H); 2,22 (s, 3H); 2,62 (m, 2H); 3,13 (m, 2H); 3,30 (m, 3H); 3,46 (3, 2H); 3,73 (s, 3H); 4,52 (m, 1H); 5,18 (dd, 1H); 6,45 (d, 1H); 6,90-7,40 (m, 8H).

Beispiel 21

Methyl-erythro-(E)-7-[3-(4-fluor-3-phenoxyphenyl)-5-isopropyl-2-methyl-1-piperidino-pyrrol-4-yl]-3,5-dihydroxy-hept-5-enoat

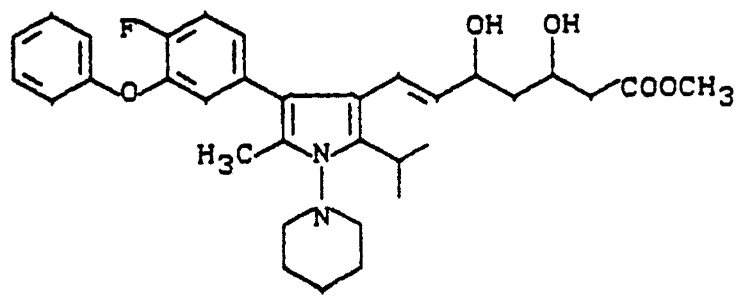

1,7 g (3 mmol) der Verbindung aus Beispiel 20 werden analog Beispiel 6 umgesetzt.
Ausbeute: 330 mg (19,5 % der Theorie).
$^1$H-NMR ($CDCl_3$): $\delta$ = 1,32 (d, 6H); 1,20-1,80 (m, 8H); 2,20 (s, 3H); 2,47 (m, 2H); 3,15 (m, 2H); 3,30 (m, 3H); 3,71 (s, 3H); 4,22 (m, 1H); 4,33 (m, 1H); 5,19 (dd, 1H); 6,40 (d, 1H); 6,90-7,20 (m, 6H); 7,30 (m, 2H).

Beispiel 22

3-(4-Fluorphenyl)-1-hexahydroazepinyl-5-isopropyl-2-phenyl-pyrrol

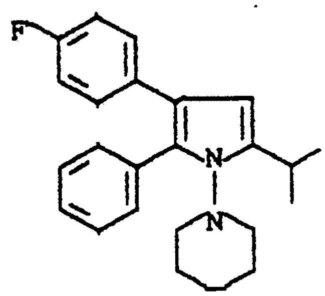

5 g (16,7 mmol) der Verbindung aus Beispiel 2 und 5,7 g (50,1 mmol) N-Amino-hexahydroazepin in 100 ml Toluol p.A. und 80 ml Dimethylformamid werden mit 1,7 ml konz. Salzsäure versetzt und 24 Stunden am Wasserabscheider gekocht. Die Mischung wird abgekühlt, mit 200 ml Essigester verdünnt und dreimal mit 1 N Salzsäure und zweimal mit gesättigter Natriumhydrogencarbonat-Lösung extrahiert. Die organische Phase wird über Magnesiumsulfat getrocknet, im Vakuum eingeengt und der Rückstand an einer Säule (Kieselgel 70-230 mesh, mit Essigester/Petrolether 1:9) chromatographiert.
Ausbeute: 560 mg (8,9 % der Theorie).
$^1$H-NMR ($CDCl_3$): $\delta$ = 1,32 (d, 6H); 1,35-1,7 (m, 8H); 3,0-3,4 (m, 5H); 6,01 (s, 1H); 6,8 (m, 2H); 7,05 (m, 2H); 7,35 (m, 5H).

Beispiel 23

(E)-3-[3-(4-Fluorphenyl)-1-hexahydroazepinyl-5-isopropyl-2-phenyl-pyrrol-4-yl]-prop-2-enal

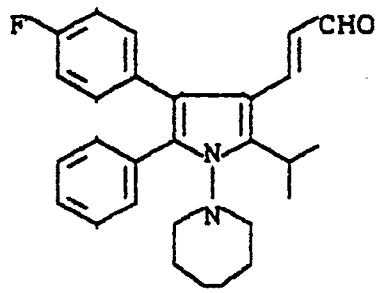

550 mg (1,5 mmol) der Verbindung aus Beispiel 22 werden analog Beispiel 4 umgesetzt.
Ausbeute: 410 mg (63,6 % der Theorie).
$^1$H-NMR ($CDCl_3$): $\delta$ = 1,28-1,7 (m, 8H); 1,5 (d, 6H); 3,08 (m, 2H); 3,28 (m, 2H); 3,58 (m, 1H); 5,7 (dd, 1H); 6,84 (m, 2H); 7,02 (m, 2H); 7,21 (m, 5H); 7,6 (d, 1H); 9,35 (d, 1H).

Beispiel 24

Methyl-(E)-7-[3-(4-fluorphenyl)-1-hexahydroazepinyl-5-isopropyl-2-phenyl-pyrrol-4-yl]-5-hydroxy-3-oxo-hept-6-enoat

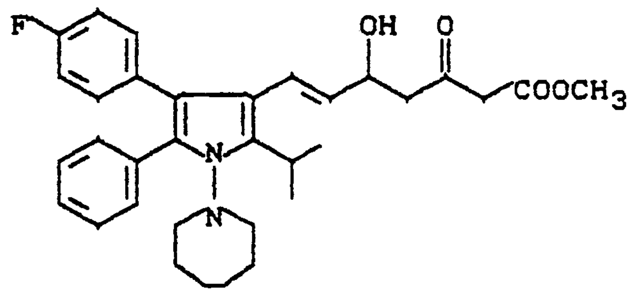

400 mg (0,93 mmol) der Verbindung aus Beispiel 23 werden analog Beispiel 5 umgesetzt.
Ausbeute: 187 mg (36,8 % der Theorie).
$^1$H-NMR ($CDCl_3$): $\delta$ = 1,42 (d, 6H); 1,30-1,80 (m, 8H); 2,58 (m, 2H); 3,08 (m, 2H); 3,30 (m, 2H); 3,42 (m, 1H); 3,48 (s, 2H); 3,73 (s, 3H); 4,53 (m, 1H); 5,08 (dd, 1H); 6,68 (d, 1H); 6,82 (m, 2H); 7,04 (m, 2H); 7,22 (m, 5H).

Beispiel 25

Methyl-erythro-(E)-7-[3-(4-fluorphenyl)-1-hexahydroazepinyl-5-isopropyl-2-phenyl-pyrrol-4-yl]-3,5-dihydroxy-hept-6-enoat

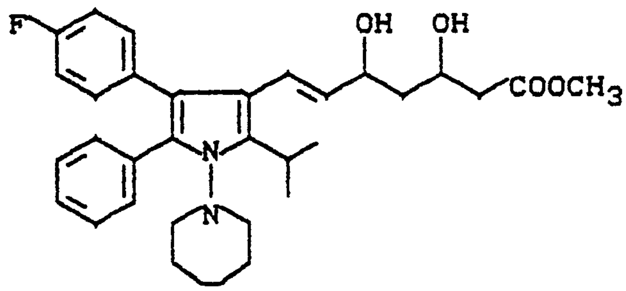

180 mg (0,33 mmol) der Verbindung aus Beispiel 24 werden analog Beispiel 6 umgesetzt.
Ausbeute: 130 mg (72,2 % der Theorie).
$^1$H-NMR ($CDCl_3$): $\delta$ = 1,43 (d, 6H); 1,20-1,80 (m, 10H); 2,74 (m, 2H); 3,10 (m, 2H); 3,31 (m, 2H); 3,41 (m, 1H); 3,72 (s, 3H); 4,19 (m, 1H); 4,33 (m, 1H); 5,12 (dd, 1H); 6,62 (d, 1H); 6,80 (m, 2H); 7,03 (m, 2H); 7,22 (m, 5H).

Beispiel 26

3-(4-Fluorphenyl)-5-isopropyl-1-(4-methylpiperidino)-2-phenyl-pyrrol

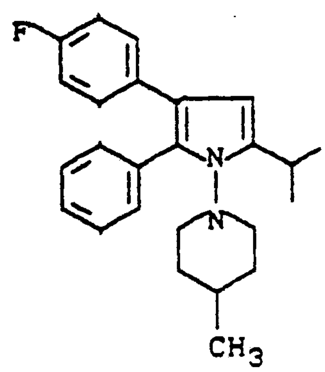

10,1 g (34 mmol) der Verbindung aus Beispiel 2 und 11,6 g (102 mmol) N-Amino-4-methyl-piperidin in 100 ml Toluol p.A. und 50 ml Dimethylformamid werden mit 3,4 ml konz. Salzsäure versetzt und 24 Stunden am Wasserabscheider gekocht. Die Mischung wird abgekühlt, mit 200 ml Essigester verdünnt und dreimal mit 1 N Salzsäure und zweimal mit gesättigter Natriumhydrogencarbonat-Lösung extrahiert. Die organische Phase wird über Magnesiumsulfat getrocknet, im Vakuum eingeengt und der Rückstand an einer Säule (Kieselgel 70-230 mesh, mit Essigester/Petrolether 5:95) chromatographiert.
Ausbeute: 1,9 g (14,8 % der Theorie).

Beispiel 27

E-(3)-[3-(4-Fluorphenyl)-5-isopropyl-1-(4-methylpiperidino)-2-phenyl-pyrrol-4-yl]-prop-2-enal

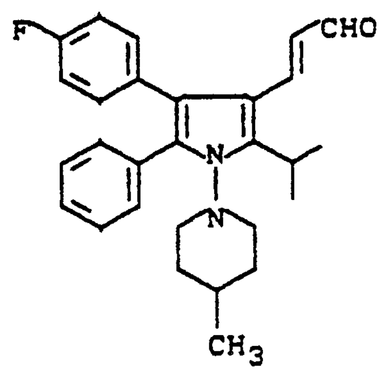

1,9 g (5,1 mmol) der Verbindung aus Beispiel 26 werden analog Beispiel 4 umgesetzt.
Ausbeute: 1,2 g (54,5 % der Theorie).
$^1$H-NMR ($CDCl_3$): $\delta$ = 0,88 (d, 3H); 1,0-1,70 (m, 5H); 1,48 (d, 6H); 2,97 (m, 2H); 3,12 (m, 2H); 3,56 (m, 1H); 5,77 (dd, 1H); 6,82 (m, 2H); 7,0 (m, 2H); 7,27 (m, 5H); 7,52 (d, 1H); 9,38 (d, 1H).

Beispiel 28

Methyl-(E)-7-[3-(4-fluorphenyl)-5-isopropyl-1-(4-methylpiperidino)-pyrrol-4-yl]-5-hydroxy-3-oxo-hept-6-enoat

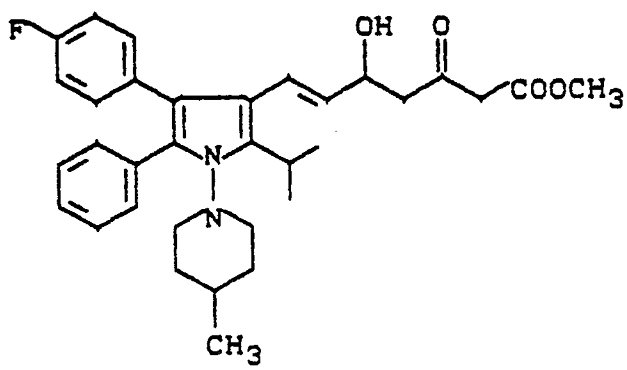

1,2 g (2,8 mmol) der Verbindung aus Beispiel 27 werden analog Beispiel 5 umgesetzt.
Ausbeute: 850 mg (55,6 % der Theorie).
$^1$H-NMR ($CDCl_3$): $\delta$ = 0,87 (d, 3H); 1,0-1,70 (m, 5H); 1,42 (d, 6H); 2,62 (m, 2H); 2,91 (m, 2H); 3,09 (m, 2H); 3,40 (m, 1H); 3,48 (s, 2H); 3,73 (s, 3H); 4,55 (m, 1H); 5,18 (dd, 1H); 6,52 (d, 1H); 6,78 (m, 2H); 6,99 (m, 2H); 7,25 (m, 5H).

Beispiel 29

Methyl-erythro-(E)-7-[3-(4-fluorphenyl)-5-isopropyl-1-(4-methylpiperidino)-pyrrol-4-yl]-3,5-dihydroxy-hept-6-enoat

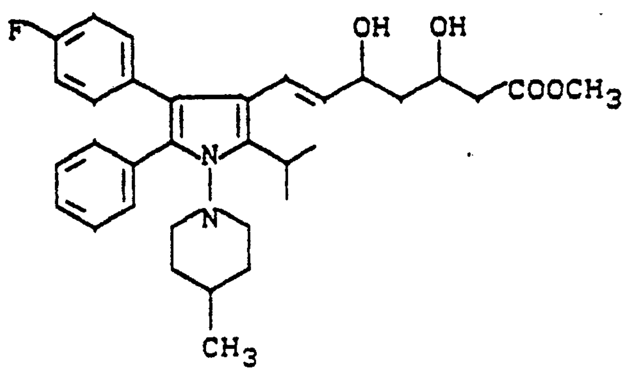

830 mg (1,7 mmol) der Verbindung aus Beispiel 28 werden analog Beispiel 6 umgesetzt.
Ausbeute: 620 mg (66,5 % der Theorie).
$^{1}$H-NMR ($CDCl_3$): $\delta$ = 0,87 (d, 3H); 1,0-1,70 (m, 7H); 1,39 (d, 6H); 2,47 (m, 2H); 2,90 (m, 2H); 3,12 (m, 2H); 3,39 (m, 1H); 3,72 (s, 3H); 4,22 (m, 1H); 4,38 (m, 1H); 5,22 (dd, 1H); 6,48 (d, 1H); 6,76 (m, 2H); 6,98 (m, 2H); 7,25 (m, 5H).

## Anwendungsbeispiel

### Beispiel 30

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Cholestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m $K_xH_y$ Phosphat, 0,03 m Ethylendiamintetraessigsäure, 0,002 m Dithiothreit (SPE)-Puffer pH 7,2, homogenisiert. Anschließend wurde 15 Minuten bei 15 000* g zentrifugiert und das Sediment verworfen. Der Überstand wurde 75 Minuten bei 100 000 g sedimentiert. Das Pellet wird in 1/4 Volumen SPE-Puffer aufgenommen, nochmals homogenisiert und anschließend erneut 60 Minuten bei 100.000 g zentrifugiert. Das Pellet wird mit der 5-fachen Menge ihres Volumes SPE-Puffer aufgenommen, homogenisiert und bei -78 °C eingefroren und gelagert ( = Enzymlösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37 °C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase, 35 µMol $K_xH_y$-Phosphat pH 7,2, 20 µl Enzympräparation und 56 nMol 3-Hydroxy-3-methyl-glutaryl Coenzym A (Glutaryl-3-$^{14}$C) 100 000 dpm.

Man inkubierte 60 Minuten bei 37 °C und stoppte die Reaktion durch Zusatz von 300 µl 0,24 m HCl ab. Nach einer Nachinkubation von 60 Minuten bei 37 °C wurde der Ansatz zentrifugiert und 600 µl des Überstandes auf eine 0,7 x 4 cm mit Biorex $^{(R)}$ 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt und im LKB-Scintillationszähler gezählt. $IC_{50}$-Werte wurden durch Auftrag der prozentualen Hemmung gegen die Konzentration der Verbindung im Test durch Intrapolation bestimmt. Zur Bestimmung der relativen inhibitorischen Potenz wurde der $IC_{50}$-Wert der Referenzsubstanz Mevinolin als 100 gesetzt und mit dem simultan bestimmten $IC_{50}$-Wert der Testverbindung verglichen.

### Beispiel 31

Die subchronische Wirkung der erfindungsgemäßen Verbindungen auf die Blutcholesterinwerte von Hunden wurde in mehrwöchigen Fütterungsexperimenten geprüft. Dazu wurde die zu untersuchende

Substanz über einen mehrwöchigen Zeitraum einmal täglich in einer Kapsel gesunden Beagle Hunden zusammen mit dem Futter p.o. gegeben. Dem Futter war außerdem während der gesamten Versuchsperiode, d.h. vor, während und nach der Applikationsperiode der zu untersuchenden Substanz, Colestyramin (4 g/100 g Futter) als Gallensäuresequestrant beigemischt. Zweimal wöchentlich wurde den Hunden venöses Blut entnommen und das Serumcholesterin enzymatisch bestimmt. Die Serumcholesterinwerte während der Applikationsperiode wurden mit den Serumcholesterinwerten vor der Applikationsperiode (Kontrolle) verglichen.

## Ansprüche

1. N-substituierte N-amino-pyrrole der Formel

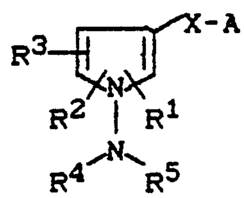

(I)

in welcher

$R^1$ - für Cycloalkyl steht, oder

- für Alkyl steht, das substituiert sein kann durch Halogen, Cyano, Hydroxy, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^6R^7$,

worin

$R^6$, $R^7$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^6R^7$ substituiert sein kann, worin

$R^6$ und $R^7$ die oben angegebene Bedeutung haben,

oder

- für Aryl steht, das bis zu 5-fach gleich oder verschieden substituiert sein kann durch Alkyl, Hydroxyalkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^6R^7$,

worin

$R^6$ und $R^7$ die oben angegebene Bedeutung haben,

$R^3$ - für Wasserstoff oder

- für Cycloalkyl steht, oder

- für Alkyl steht, das substituiert sein kann durch Halogen, Cyano, Hydroxy, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^6R^7$,

worin

$R^6$, $R^7$ - die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

- für Heteroaryl steht, das bis zu 3-fach gleich oder verschieden durch Halogen, Alkyl, Alkoxy, Alkylthio, Alkylsulfonyl, Aryl, Aryloxy, Aryl thio, Arylsulfonyl, Trifluormethyl, Trifluormethoxy, Trifluormethylthio, Alkoxycarbonyl oder durch eine Gruppe der Formel $-NR^6R^7$ substituiert sein kann,
worin
$R^6$ und $R^7$ die oben angegebene Bedeutung haben,
oder
- für Aryl steht, das bis zu 5-fach gleich oder verschieden substituiert sein kann durch Alkyl, Hydroxyalkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^6R^7$,
worin
$R^6$ und $R^7$ die oben angegebene Bedeutung haben,
$R^4$ und $R^5$ gleich oder verschieden sind und für
- Wasserstoff,
- für Cycloalkyl stehen, oder
- für Alkyl stehen, das substituiert sein kann durch Halogen, Hydroxy, Alkylthio, Acyl oder durch eine Gruppe der Formel $-NR^6R^7$,
worin
$R^6$, $R^7$ - die oben angegebene Bedeutung haben,
oder durch Carbamoyl, Heteroaryl, Aryl, Aryloxy, Arylthio, wobei die Heteroaryl- und Arylreste der letztgenannten Substituenten bis zu 3-fach gleich oder verschieden durch Halogen, Trifluormethyl, Alkyl, Alkoxy, Alkylthio oder Alkylsulfonyl substituiert sein können,
oder
- für Heteroaryl stehen, das bis zu 2-fach gleich oder verschieden durch halogen, Alkyl, Alkoxy, Alkylthio, Alkylsulfonyl, Aryl, Trifluormethyl, Alkoxycarbonyl oder durch eine Gruppe der Formel $-NR^6R^7$ substituiert sein kann,
worin
$R^6$ und $R^7$ die oben angegebene Bedeutung haben,
oder
- für Aryl stehen, das bis zu 2-fach gleich oder verschieden substituiert sein kann durch Alkyl, Hydroxyalkyl, Alkoxy, Alkylthio, Alkylsulfonyl, Halogen, Cyano, Nitro, Trifluormethyl, Alkoxycarbonyl, Carbamoyl oder durch eine Gruppe der Formel $-NR^6R^7$,
worin
$R^6$ und $R^7$ die oben angegebene Bedeutung haben,
oder
- $R^4$ und $R^5$ gemeinsam mit dem N-Atom einen 4-bis 8-gliedrigen Heterocyclus bilden, der ein weiteres Heteroatom Y enthalten kann, wobei
Y für S, O oder $N-R^8$ steht, wobei
$R^8$ für Alkyl, Aryl, Aralkyl, Acyl, Alkylsulfonyl, Arylsulfonyl bedeuten kann,
wobei dieser Heterocyclus durch $R^9$ substituiert sein kann, wobei
$R^9$ - für Cycloalkyl steht, oder
- für Alkyl steht, das substituiert sein kann durch Halogen, Hydroxy, Alkoxy, Alkylthio, Acyl oder durch eine Gruppe der Formel $-NR^6R^7$,
worin
$R^6$, $R^7$ - die oben angegebene Bedeutung haben,
oder durch Carbamoyl, Heteroaryl, Aryl, Aryloxy, Arylthio, wobei die Heteroaryl- und Arylreste der letztgenannten Substituenten bis zu 2-fach gleich oder verschieden durch Halogen, Trifluormethyl, Alkyl, Alkoxy, Alkylthio oder Alkylsulfonyl substituiert sein können,
oder
- für Heteroaryl steht, das bis zu 2-fach gleich
oder verschieden durch Halogen, Alkyl, Alkoxy, Alkylsulfonyl, Aryl, Trifluormethyl, Alkoxycarbonyl oder durch eine Gruppe der Formel $-NR^6R^7$ substituiert sein kann,
worin
$R^6$ und $R^7$ die oben angegebene Bedeutung haben,
oder
- für Aryl steht, das bis zu 2-fach gleich oder verschieden substituiert sein kann durch Alkyl, Hydroxyalkyl, Alkoxy, Alkylthio, Alkylsulfonyl, Halogen, Cyano, Nitro, Trifluormethyl, Alkoxycarbonyl, Carbamoyl oder durch eine Gruppe der Formel $-NR^6R^7$,
worin
$R^6$ und $R^7$ die oben angegebene Bedeutung haben,

X - für eine Gruppe der Formel $-CH_2-CH_2-$ oder $-CH=CH-$steht,
und
A = für eine Gruppe der Formel

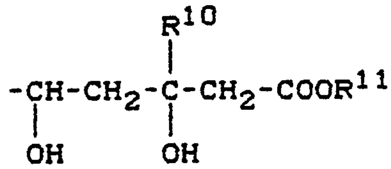
oder
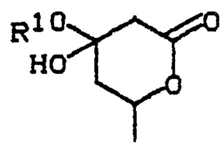
steht,

worin
$R^{10}$ - Wasserstoff oder Alkyl bedeutet
und
$R^{11}$ - Wasserstoff,
- einen Alkyl-, Aryl- oder einen Aralkylrest, oder
- ein Kation bedeutet.

2. N-substituierte N-Amino-pyrrole nach Anspruch 1,
worin
$R^1$ - für Cyclopropyl, Cyclopentyl oder Cyclohexyl steht, oder
- für Niedrigalkyl steht, das substituiert sein kann durch Fluor, Chlor, Brom, Cyano, Hydroxy, Niedrigalkoxy, Niedrigalkylthio, Niedrigalkylsulfonyl, Trifluormethyl, Trifluormethoxy, Trifluormethylsulfonyl, Niederalkoxycarbonyl, Benzoyl, Niedrigalkylcarbonyl, durch eine Gruppe der Formel $-NR^6R^7$,
worin
$R^6$ und $R^7$ gleich oder verschieden sind und Niedrigalkyl, Phenyl, Benzyl, Acetyl, Benzoyl, Phenylsulfonyl oder Niederalkylsulfonyl bedeuten,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niedrigalkyl, Niedrigalkoxy, Trifluormethyl, oder Trifluormethoxy substituiert sein können,
$R^2$ - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, die bis zu 2-fach gleich oder verschieden substituiert sein können durch Fluor, Chlor, Brom, Niedrigalkyl, Niedrigalkoxy, Phenyl, Phenoxy, Trifluormethyl, Trifluormethoxy oder Niedrigalkoxycarbonyl, oder
- für Phenyl oder Naphthyl steht, die bis zu 4-fach gleich oder verschieden substituiert sein können durch Niedrigalkyl, Niedrigalkoxy, Niedrigalkylthio, Niedrigalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niedrigalkoxycarbonyl oder durch eine Gruppe der Formel $-NR^6R^7$,
wobei
$R^6$ und $R^7$ die oben angegebene Bedeutung haben,
$R^3$ - für Wasserstoff oder
- für Cyclopropyl, Cyclopentyl oder Cyclohexyl steht oder
- für Niedrigalkyl, das substituiert sein kann durch Fluor, Chlor, Cyano, Hydroxy, Niedrigalkoxy, Niedrigalkylthio, Niedrigalkylsulfonyl, Trifluormethyl, Niedrigalkoxycarbonyl, Benzoyl, Niedrigalkylcarbonyl oder durch eine Gruppe der Formel $-NR^6R^7$,
worin
$R^6$ und $R^7$ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylsulfonyl, Benzyloxy, Phenylethoxy, wobei die genannten Heteroaryl- und Arylreste bis zu 2-fach gleich oder verschieden durch Fluor, Chlor, Niedrigalkyl, Niedrigalkoxy, Trifluormethyl substituiert sein können,
- für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothia zolyl, Benzoxazolyl oder Benzimidazolyl das bis zu 2-fach gleich oder ver-schieden substituiert sein kann durch Fluor, Chlor, Niedrigalkyl, Niedrigalkoxy, Phenyl, Phenoxy, Trifluormethyl oder Niederalkoxycarbonyl, oder

- Phenyl oder Naphthyl bedeutet, das bis zu 2-fach gleich oder verschieden substituiert sein kann durch Niedrigalkyl, Niedrighydroxyalkyl, Niederalkoxy, Phenyl, Phenyloxy, Benzyl, Benzyloxy, Phenethyl, Phenylethoxy, Fluor, Chlor, Cyano, Trifluormethyl, Niedrigalkoxycarbonyl oder durch eine Gruppe der Formel $-NR^6R^7$,

wobei

$R^6$ und $R^7$ die oben angegebene Bedeutung haben

$R^4$ und $R^5$ gleich oder verschieden sein können und

- für Wasserstoff,

- für Cyclopropyl, Cyclopentyl oder Cyclohexyl stehen

- oder für Niedrigalkyl stehen, das substituiert sein kann durch Fluor, Chlor, Hydroxy, Niedrig alkoxy, Trifluormethyl, Benzoyl, oder durch eine Gruppe der Formel $-NR^6R^7$, worin

$R^6$ und $R^7$ die oben angegebene Bedeutung haben,

oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Benzyloxy, Phenylethoxy, wobei die genannten Heteroaryl-und Arylreste bis zu 2-fach gleich oder verschieden durch Fluor, Chlor, Niedrigalkyl, Niedrigalkoxy, Trifluormethyl substituiert sein können,

- für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl stehen, die bis zu 2-fach gleich oder verschieden substituiert sein können durch Fluor, Chlor, Niedrigalkyl, Niedrigalkoxy, Phenyl, Phenoxy, Trifluormethyl oder Niedrigalkoxycarbonyl, oder

- für Phenyl oder Naphthyl stehen, die bis zu 2-fach gleich oder verschieden substituiert sein können durch Niedrigalkyl, Niedrigalkoxy, Niedrigalkylthio, Niedrigalkylsulfonyl, Phenyl, Phenyloxy, Phenylthio, Phenylsulfonyl, Benzyl, Benzyloxy, Phenethyl, Phenylethoxy, Fluor, Chlor, Cyano, Trifluormethyl, Niedrigalkoxycarbonyl oder durch eine Gruppe der Formel $-NR^6R^7$,

wobei

$R^6$ und $R^7$ die oben angegebene Bedeutung haben

oder

$R^4$ und $R^5$ gemeinsam mit dem N-Atom einen 5- bis 7-gliedrigen Heterocyclus bilden, der ein weiteres Heteroatom Y enthalten kann wobei

Y für S, O oder $N-R^8$ steht, wobei

$R^8$ - für Niederalkyl, Phenyl, Benzyl, Niedrigalkylsulfonyl, Phenylsulfonyl, Benzoyl oder Acetyl steht,

wobei dieser Heterocyclus durch $R^9$ substituiert sein kann, wobei

$R^9$ - für Cyclopropyl, Cyclopentyl oder Cyclohexyl stehen kann, oder

- für Niedrigalkyl steht, das substituiert sein kann durch Fluor, Chlor, Hydroxy, Niedrigalkoxy, Benzoyl, Niedrigalkyl carbonyl oder durch eine Gruppe der Formel $-NR^6R^7$,

worin

$R^6$, $R^7$ - die oben angegebene Bedeutung haben,

oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylsulfonyl, Benzyloxy oder Phenylethoxy, wobei die genannten Heteroaryl- und Arylreste bis zu 2-fach gleich oder verschieden durch Fluor, Chlor, Hydroxyalkyl, Niedrigalkyl, Niedrigalkoxy, Trifluormethyl substituiert sein können,

- für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl stehen, die bis zu 2-fach gleich oder verschieden substituiert sein können durch Fluor, Chlor, Niedrigalkyl, Niedrigalkoxy, Phenyl, Phenoxy, Trifluormethyl oder Niedrigalkoxycarbonyl, oder

- für Phenyl steht, das bis zu 2-fach gleich oder verschieden substituiert sein kann durch Niedrigalkyl, Niedrigalkoxy, Niedrigalkylsulfonyl, Phenyl, Phenyloxy, Phenylsulfonyl, Benzyl, Benzyloxy, Phenethyl, Phenylethoxy, Fluor, Chlor, Cyano, Trifluormethyl, Niedrigalkoxycarbonyl oder durch eine Gruppe der Formel $-NR^6R^7$,

wobei

$R^6$ und $R^7$ die oben angegebene Bedeutung haben

X - für eine Gruppe der Formel $-CH_2-CH_2-$ oder $-CH=CH-$steht,

A - für eine Gruppe der Formel

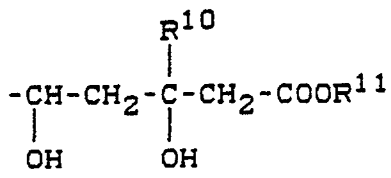 oder 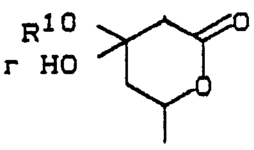

steht, worin

$R^{10}$ - Wasserstoff oder Niedrigalkyl bedeutet, und

$R^{11}$- einen $C_1$-$C_6$-Alkylrest, einen $C_6$-$C_{12}$-Alkylrest oder einen $C_7$-$C_{10}$-Alkylrest oder

- ein physiologisch verträgliches Kation bedeutet.

3. Substituierte N-Amino-pyrrole nach den Ansprüchen 1 und 2, worin

R' - für Cyclopropyl, Cyclopentyl oder Cyclohexyl steht,oder

- für Methyl, Ethyl, Propyl, Isopropyl, Butyl, sec.-Butyl oder tert.Butyl steht, die substituiert sein können durch Fluor, Chlor, Hydroxy, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylsulfonyl, Benzyloxy, Benzylthio oder Benzylsulfonyl,

$R^2$ - für Pyridyl, Pyrimidyl, Chinolyl oder Isochinolyl steht, die durch Fluor, Chlor, Methyl, Methoxy oder Trifluormethyl substituiert sein können, oder

- für Phenyl steht, das bis zu 2-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Methoxycarbonyl, Ethoxycarbonyl, Propoxycarbonyl, Isopropoxycarbonyl, Butoxycarbonyl, Isobutoxycarbonyl oder tert.-Butoxycarbonyl,

$R^3$ - Wasserstoff, Cyclopropyl, Cyclopentyl oder Cyclohexyl bedeutet oder

- Methyl, Ethyl, Propyl, Isopropyl, Butyl, Isobutyl, tert.Butyl, Pentyl, Isopentyl, Hexyl oder Isohexyl bedeutet, das substituiert sein kann durch Fluor, Chlor, Cyano, Hydroxy, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe $-NR^6R^7$,

wobei

$R^6$ und $R^7$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,

$R^4$ und $R^5$ gleich oder verschieden sein können und

- für Wasserstoff, Cyclopropyl, Cyclopentyl oder Cyclohexyl stehen, oder

- für Methyl, Ethyl, Propyl, Isopropyl, Butyl, Isobutyl, tert.Butyl, Pentyl, Isopentyl, Hexyl oder Isohexyl stehen, die substituiert sein können durch Fluor, Chlor, Hydroxy, Methoxy, Ethoxy, Methylthio, Ethylthio, Propylthio, Isopropylthio, Butylthio, Isobutylthio, tert.-Butylthio, Benzoyl, Acetyl, Ethylcarbonyl, oder durch eine Gruppe $-NR^6R^7$,

wobei

$R^6$ und $R^7$ die oben angegebene Bedeutung haben,

oder durch Pyrrolyl, Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl substituiert sein können, oder

- für Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzothiazolyl stehen, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl,

Isobutoxycarbonyl oder tert.-Butoxycarbonyl substituiert sein können, oder

- für Phenyl stehen, das bis zu 2-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Phenyl, Phenoxy, Phenylthio, Phenylsulfonyl, Benzyl, Benzyloxy, Fluor, Chlor, Cyano, Hydroxy, Trifluormethyl, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^6R^7$ substituiert sein kann,

wobei

$R^6$ und $R^7$ die oben angegebene Bedeutung haben,

oder

$R^4$ und $R^5$ gemeinsam mit dem N-Atom einen 5- bis 7-gliedrigen Heterocyclus bilden, der ein weiteres Heteroatom Y enthalten kann, wobei

Y für S, O oder $N-R^8$ steht,

wobei $R^8$

Methyl, Ethyl, Propyl, Isopropyl, Butyl, Isobutyl, tert.-Butyl, Phenyl, Benzyl, Acetyl, Methylsulfonyl, Ethylsulfonyl, Propylsulfonyl, Isopropylsulfonyl oder Phenylsulfonyl bedeuten kann

wobei dieser Heterocyclus durch $R^9$ substituiert sein kann

wobei $R^9$

- für Cyclopropyl, Cyclopentyl oder Cyclohexyl steht oder
- für Methyl, Ethyl, Propyl, Isopropyl, Butyl, Isobutyl, tert.-Butyl, Pentyl, Isopentyl, Hexyl oder Isohexyl steht, die substituiert sein können durch Fluor, Chlor, Hydroxy, Methoxy, Ethoxy, Trifluormethyl, Benzoyl, Butyl, Ethylcarbonyl oder durch eine Gruppe $-NR^6R^7$

wobei $R^6$ und $R^7$ die oben angegebene Bedeutung haben

- für Pyridyl, Pyrimidyl oder Benzimidazolyl steht, die bis zu 2-fach gleich oder verschieden substituiert sein können durch Fluor, Chlor, Methyl, Ethyl, Methoxy, Ethoxy, Phenyl, Phenoxy oder Trifluormethyl
- für Phenyl steht, das bis zu 2-fach gleich oder verschieden substituiert sein kann durch Methyl, Ethyl, Propyl, tert.-Butyl, Methylsulfonyl, Ethylsulfonyl, Propylsulfonyl, Phenyl, Phenyloxy, Phenylsulfonyl, Benzyl, Benzyloxy, Phenethyl, Phenylethoxy, Fluor, Chlor, Cyano, Trifluormethyl oder durch eine Gruppe der Formel $-NR^6R^7$,

wobei

$R^6$ und $R^7$ die oben angegebene Bedeutung haben,

X - für eine Gruppe der Formel $-CH_2-CH_2-$ oder $-CH=CH-$steht,

A - für eine Gruppe der Formel

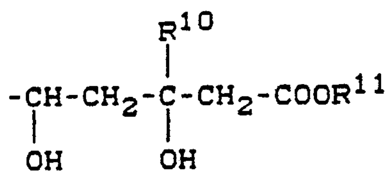 oder 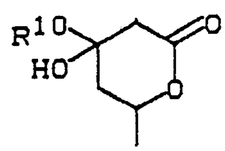 steht,

worin

$R^{10}$ - Wasserstoff, Methyl, Ethyl, Propyl, Isopropyl, Butyl, Isobutyl oder tert.-Butyl bedeutet,

und

$R^{11}$- Wasserstoff, Methyl, Ethyl, Propyl, Isopropyl, Butyl, Isobutyl, tert.Butyl oder Benzyl bedeutet, oder ein Natrium-, Kalium-, Calcium -, oder Magnesium- oder Ammoniumion bedeutet.

4. N-substiutierte N-Amino-pyrrole nach den Ansprüchen 1 bis 3 zur therapeutischen Behandlung.

5. Verfahren zur Herstellung von N-substituierten N-Amino-pyrrolen der Formel

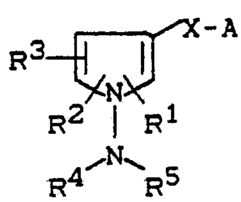

(I)

in welcher

$R^1$ - für Cycloalkyl steht, oder
- für Alkyl steht, das substituiert sein kann durch Halogen, Cyano, Hydroxy, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^6R^7$,
worin
$R^6$, $R^7$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^6R^7$ substituiert sein kann, worin
$R^6$ und $R^7$ die oben angegebene Bedeutung haben,
oder
- für Aryl steht, das bis zu 5-fach gleich oder verschieden substituiert sein kann durch Alkyl, Hydroxyalkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^6R^7$,
worin
$R^6$ und $R^7$ die oben angegebene Bedeutung haben,
$R^3$ - für Wasserstoff oder
- für Cycloalkyl steht, oder
- für Alkyl steht, das substituiert sein kann durch Halogen, Cyano, Hydroxy, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^6R^7$,
worin
$R^6$, $R^7$ - die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
oder
- für Heteroaryl steht, das bis zu 3-fach gleich oder verschieden durch Halogen, Alkyl, Alkoxy, Alkylthio, Alkylsulfonyl, Aryl, Aryloxy, Aryl thio, Arylsulfonyl, Trifluormethyl, Trifluormethoxy, Trifluormethylthio, Alkoxycarbonyl oder durch eine Gruppe der Formel $-NR^6R^7$ substituiert sein kann,
worin
$R^6$ und $R^7$ die oben angegebene Bedeutung haben,
oder
- für Aryl steht, das bis zu 5-fach gleich oder verschieden substituiert sein kann durch Alkyl, Hydroxyalkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^6R^7$,
worin
$R^6$ und $R^7$ die oben angegebene Bedeutung haben,
$R^4$ und $R^5$ gleich oder verschieden sind und für
- Wasserstoff,
- für Cycloalkyl stehen, oder
- für Alkyl stehen, das substituiert sein kann durch Halogen, Hydroxy, Alkoxy, Alkylthio, Acyl oder durch eine Gruppe der Formel $-NR^6R^7$,
worin
$R^6$, $R^7$ - die oben angegebene Bedeutung haben,
oder durch Carbamoyl, Heteroaryl, Aryl, Aryloxy, Arylthio, wobei die Heteroaryl- und Arylreste der letztgenannten Substituenten bis zu 3-fach gleich oder verschieden durch Halogen, Trifluormethyl, Alkyl, Alkoxy, Alkylthio oder Alkylsulfonyl substituiert sein können,
oder
- für Heteroaryl stehen, das bis zu 2-fach gleich oder verschieden durch Halogen, Alkyl, Alkoxy, Alkylthio,

Alkylsulfonyl, Aryl, Trifluormethyl, Alkoxycarbonyl oder durch eine Gruppe der Formel $-NR^6R^7$ substituiert sein kann,
worin
$R^6$ und $R^7$ die oben angegebene Bedeutung haben,
oder
- für Aryl stehen, das bis zu 2-fach gleich oder verschieden substituiert sein kann durch Alkyl, Hydroxyalkyl, Alkoxy, Alkylthio, Alkyl sulfonyl, Halogen, Cyano, Nitro, Trifluormethyl, Alkoxycarbonyl, Carbamoyl oder durch eine Gruppe der Formel $-NR^6R^7$,
worin
$R^6$ und $R^7$ die oben angegebene Bedeutung haben,
oder
- $R^4$ und $R^5$ gemeinsam mit dem N-Atom einen 4-bis 8-gliedrigen Heterocyclus bilden, der ein weiteres Heteroatom Y enthalten kann,
wobei
Y für S, O oder $N-R^8$ steht, wobei
$R^8$ für Alkyl, Aryl, Aralkyl, Acyl, Alkylsulfonyl, Arylsulfonyl stehen kann,
wobei dieser Heterocyclus durch $R^9$ substituiert sein kann, wobei
$R^9$ - für Cycloalkyl steht, oder
- für Alkyl steht, das substituiert sein kann durch Halogen, Hydroxy, Alkoxy, Alkylthio, Acyl oder durch eine Gruppe der Formel $-NR^6R^7$,
worin
$R^6$, $R^7$ - die oben angegebene Bedeutung haben,
oder durch Carbamoyl, Heteroaryl, Aryl, Aryloxy, Arylthio, wobei die Heteroaryl- und Arylreste der letztgenannten Substituenten bis zu 2-fach gleich oder verschieden durch Halogen, Trifluormethyl, Alkyl, Alkoxy, Alkylthio oder Alkylsulfonyl substituiert sein können,
oder
- für Heteroaryl steht, das bis zu 2-fach gleich oder verschieden durch Halogen, Alkyl, Alkoxy, Alkylthio, Alkylsulfonyl, Aryl, Trifluormethyl, Alkoxycarbonyl oder durch eine Gruppe der Formel $-NR^6R^7$ substituiert sein kann,
worin
$R^6$ und $R^7$ die oben angegebene Bedeutung haben,
oder
- für Aryl steht, das bis zu 2-fach gleich oder verschieden substituiert sein kann durch Alkyl, Hydroxyalkyl, Alkoxy, Alkylthio, Alkylsulfonyl, Halogen, Cyano, Nitro, Trifluormethyl, Alkoxycarbonyl, Carbamoyl oder durch eine Gruppe der Formel $-NR^6R^7$,
worin
$R^6$ und $R^7$ die oben angegebene Bedeutung haben,
X - für eine Gruppe der Formel $-CH_2-CH_2-$ oder $-CH=CH-$ steht,
und
A - für eine Gruppe der Formel

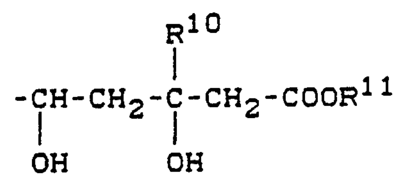 oder 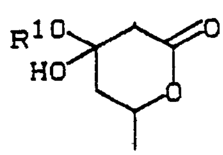 steht,

worin
$R^{10}$ - Wasserstoff oder Alkyl bedeutet
und
$R^{11}$ - Wasserstoff,
- einen Alkyl-, Aryl- oder einen Aralkylrest oder
- ein Kation bedeutet,
dadurch gekennzeichnet, daß man Ketone der allgemeinen Formel (VIII)

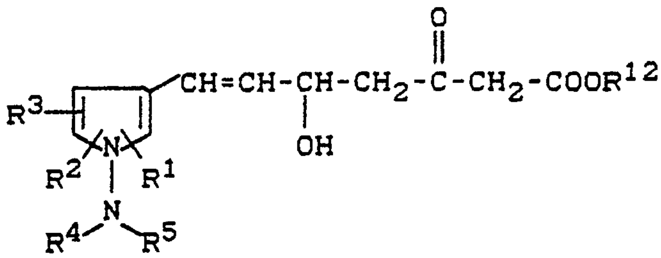

in welcher
$R^1$, $R^2$, $R^3$, $R^4$, $R^5$ die oben angegebene Bedeutung haben,
und
$R^{12}$ - für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = $-CH_2-CH_2-$) die Ethenverbindungen (X = $-CH=CH-$) nach üblichen Methoden hydriert,
und gegebenenfalls Isomeren trennt.

6. Verfahren nach Anspruch 5, dadurch gekennzeichnet, daß man die Reduktion im Temperaturbereich von -90 °C bis +30 °C ausführt.

7. Ketone der Formel

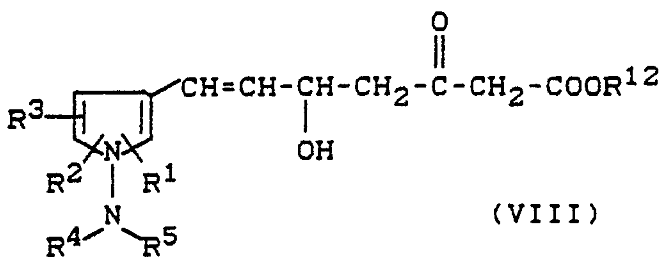

(VIII)

worin
$R^1$, $R^2$, $R^3$, $R^4$ und $R^5$ die in Anspruch 1 angegebene Bedeutung haben, und
$R^{12}$ für Alkyl steht.

8. Verfahren zur Herstellung von Ketone der Formel

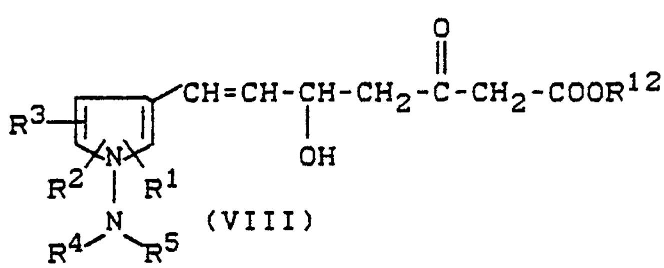

(VIII)

worin
$R^1$, $R^2$, $R^3$, $R^4$ und $R^5$ die in Anspruch 5 angegebene Bedeutung haben, und
dadurch gekennzeichnet, daß man
Aldehyde der allgemeinen Formel (IX)

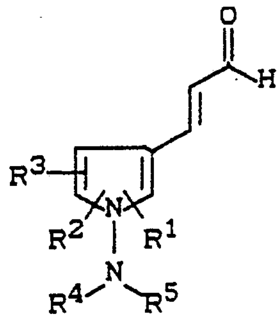

(IX)

in welcher
$R^1$, $R^2$, $R^3$, $R^4$ und $R^5$ die oben angegebene Bedeutung haben,
in inerten Lösemitteln mit Acetessigester der allgemeinen Formel (X)

$H_3C-\overset{O}{\overset{\|}{C}}-CH_2-COOR^{12}$ (X)

in welcher
$R^{12}$ die oben angegebene Bedeutung hat,
in Anwesenheit von Basen umsetzt.

9. Aldehyde der Formel

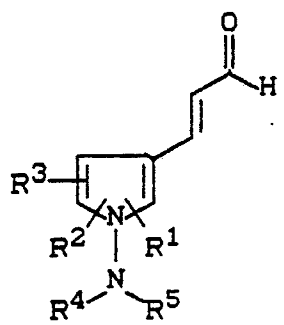

(IX)

in welcher
$R^1$, $R^2$, $R^3$, $R^4$ und $R^5$ die in Anspruch 1 angegebene Bedeutung haben, und

10. Verfahren zur Herstellung von Aldehyden der Formel

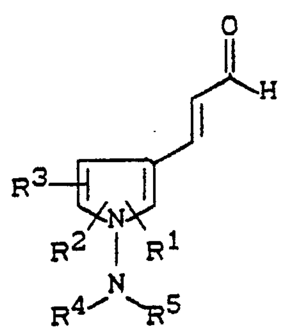

(IX)

in welcher
$R^1$, $R^2$, $R^3$, $R^4$ und $R^5$ die in Anspruch 5 angegebene Bedeutung haben, und
dadurch gekennzeichnet, daß man
Pyrrole der allgemeinen Formel (XI)

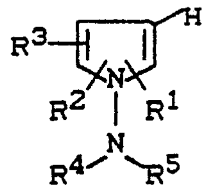

(XI)

in welcher
$R^1$, $R^2$, $R^3$, $R^4$ und $R^5$ die oben angegebene Bedeutung haben,
in inerten Lösemitteln mit Anwesenheit von Säurehalogenide mit N,N-Dialkylaminoacrolein der Formel (XII)

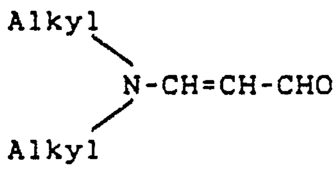

(XII)

worin
Alkyl für einen geradkettigen oder verzweigten Kohlenwasserstoffrest ()$C_1$ bis $C_4$) steht,
umsetzt.

11. Arzneimittel, enthaltend N-substituierte N-Amino-pyrrole nach den Ansprüchen 1 bis 3.

12. Arzneimittel nach Anspruch 11, dadurch gekennzeichnet, daß es 0,5 bis 90 Gew.-% an N-substituierten N-Amino-pyrrolen, bezogen auf die Gesamtmischung, enthält.

13. Verwendung von N-substituierten N-Amino-pyrrolen nach den Ansprüchen 1 bis 3 zur Behandlung von Krankheiten.

14. Verwendung von N-substituierten N-Amino-pyrrolen nach den Ansprüchen 1 bis 3 zur Herstellung von Arzneimitteln.

15. Verwendung nach Anspruch 14 zur Behandlung von Hyperlipoproteinämie, Lipoproteinämie oder Arteriosklerose.

Europäisches Patentamt

# EUROPÄISCHER RECHERCHENBERICHT

Nummer der Anmeldung

EP 89 10 6241

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 221 025 (SANDOZ)<br>* Ansprüche 1,9 *<br>----- | 1,15 | C 07 D 207/00<br>C 07 D 401/04<br>A 61 K 31/40 |
| | | | **RECHERCHIERTE SACHGEBIETE (Int. Cl.4)** |
| | | | C 07 D 207/00<br>C 07 D 401/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 16-06-1989 | CASADO Y MARTIN DE MERCA |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)